(19) Europäisches Patentamt
European Patent Office
Office européen des brevets

(11) **EP 3 871 683 A1**

(12) **DEMANDE DE BREVET EUROPEEN**

(43) Date de publication:
01.09.2021 Bulletin 2021/35

(51) Int Cl.:
*A61K 35/742* (2015.01)     *A61P 1/12* (2006.01)
*A61K 9/00* (2006.01)     *A23L 33/135* (2016.01)

(21) Numéro de dépôt: 21158882.7

(22) Date de dépôt: 24.02.2021

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR
Etats d'extension désignés:
BA ME
Etats de validation désignés:
KH MA MD TN

(30) Priorité: 25.02.2020 FR 2001857

(83) Déclaration conformément à la règle 32(1) CBE
(solution de l'expert)

(71) Demandeurs:
• Université de Paris
75006 Paris (FR)

• Institut National de la Santé et de la Recherche
Médicale
75013 Paris (FR)
• ASSISTANCE PUBLIQUE,
HOPITAUX DE PARIS
75004 Paris (FR)

(72) Inventeurs:
• AIRES, Julio
91330 YERRES (FR)
• BARBUT, Frédéric
75020 PARIS (FR)
• FERRARIS, Laurent
94000 Créteil (FR)

(74) Mandataire: Fidal Innovation
4-6 avenue d'Alsace
92400 Courbevoie (FR)

(54) **SOUCHE NON TOXINOGENE DE CLOSTRIDIOIDES DIFFICILE POUR SON UTILISATION DANS LA PREVENTION ET/OU LE TRAITEMENT D'UNE INFECTION ASSOCIEE A CLOSTRIDIOIDES DIFFICILE ET/OU DES RECIDIVES ASSOCIEES**

(57) La présente invention concerne une souche non toxinogène de *Clostridioides difficile* pour son utilisation dans la prévention et/ou le traitement des infections associées à *Clostridioides difficile* et/ou récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847).

**EP 3 871 683 A1**

**Description**

**Domaine technique**

La présente invention concerne une souche non toxinogène de *Clostridioides difficile* pour son utilisation dans la prévention et/ou le traitement d'une infection associée à *Clostridioides difficile* et/ou des récidives associées, ainsi qu'une composition pharmaceutique la comprenant.

**Art antérieur**

*Clostridioides difficile* (également appelé *Clostridium difficile*) est un bacille à Gram positif anaérobie sporulé capable de produire des toxines (toxines A et B). Les souches qui ne produisent pas les toxines sont considérées comme non pathogènes, non toxinogènes (NTCD). Aujourd'hui, *Clostridioides difficile* représente chez l'adulte la principale étiologie des diarrhées qui surviennent au cours ou au décours d'une antibiothérapie (DAA). Les présentations cliniques des infections à *Clostridioides difficile* (ICD) vont de la diarrhée simple, spontanément résolutive, à la colite pseudo-membraneuse (CPM) pouvant se compliquer de mégacôlon toxique, perforation voire choc septique. Les principaux facteurs de risque d'ICD sont liés soit à l'antibiothérapie ou tout autre facteur modifiant le microbiote intestinal, soit à l'hôte (âge > 65 ans, sévérité d'une maladie sous-jacente, les affections digestives chroniques, l'immunodépression, les antécédents d'ICD, la durée de l'hospitalisation).

L'épidémiologie des infections à *Clostridioides difficile* a considérablement évolué ces quinze dernières années. Aux États-Unis, 453 000 cas et 29 300 décès liés aux ICD sont recensés chaque année. *Clostridioides difficile* représente en fréquence la première cause d'infections associées aux soins (IAS). En Europe, l'ECDC (European Center for Disease Prévention and Control) estime à 124 000 le nombre annuel d'ICD et à 3% la mortalité attribuable. En France, on estime à près de 20 000 par an le nombre de patients hospitalisés avec un diagnostic d'ICD. Une étude récente suggère que le risque de décès des patients ayant une ICD est 1,58 fois plus élevé comparé à celui de témoins appariés sur un score de propension tenant compte des comorbidités et de l'âge des patients. L'impact des ICD en termes de coût est élevé : celui-ci a été estimé pour l'Union Européenne (UE) à environ 3 milliards d'euros par an. En France ce surcoût est de 9 024 euros par épisode.

Le traitement actuel de l'ICD repose sur l'antibiothérapie (métronidazole, vancomycine ou fidaxomicine *per os*). Toutefois, de nombreux pays ont rapporté une augmentation de l'incidence et de la sévérité des ICD, ainsi qu'une diminution d'efficacité des traitements habituels (vancomycine et métronidazole). Ceci peut s'expliquer d'une part, par le fait que le traitement antibiotique entretient la dysbiose intestinale, principal facteur de risque d'ICD et favorise l'apparition de récidives. D'autre part, les traitements antibiotiques participent à l'émergence de résistance aux antibiotiques.

Ainsi, de nouvelles stratégies thérapeutiques sont nécessaires pour la prise en charge et la prévention des ICD.

La transplantation de microbiote fécal (TMF), administration d'un filtrat de selles d'un donneur sain à un patient malade afin de restaurer l'effet de barrière du microbiote intestinal, est une alternative à l'antibiothérapie. En clinique, la transplantation de microbiote fécal (TMF) a montré une efficacité remarquable dans le traitement des récidives multiples d'ICD. Cependant, si la transplantation de microbiote fécal (TMF) fait partie des recommandations de l'ESCMID (European Society of Clinical Microbiology and Infectious Diseases) pour traiter les récidives multiples d'ICD (grade A et niveau de preuve scientifique I), elle n'est pas préconisée dans la prévention primaire à l'ICD. Par ailleurs certaines problématiques inhérentes à son utilisation subsistent comme la sécurité à long terme, la complexité de la sélection des donneurs, le coût du *screening* des dons de selles et l'absence d'harmonisation des pratiques.

Des essais cliniques concernant l'utilisation de souches non toxinogènes de *Clostridioides difficile* (NTCD) se limitent à deux études utilisant la souche NTCD M3. Un essai de phase I a évalué la tolérance et le taux de colonisation de la souche M3 administrée à différentes doses et durées de traitement chez 80 volontaires sains (Villano, S. A., Seiberling, M., Tatarowicz, W., Monnot-Chase, E. & Gerding, D. N. Evaluation of an Oral Suspension of VP20621, Spores of Nontoxigenic Clostridioides difficile Strain M3, in Healthy Subjects. Antimicrob. Agents Chemother. 56, 5224-5229 (2012*)*) : l'administration de $10^7$ spores de M3/jour pendant 14 jours a montré une bonne tolérance, une absence d'effet indésirable sévère et 11% d'effets indésirables modérés.

Une étude de phase II, randomisée, contrôlée, *vs* placebo a évalué l'impact d'une souche non toxinogène de *Clostridioides difficile* sur la prévention des récidives (prévention secondaire) à partir d'une cohorte de 168 patients souffrant d'un premier épisode d'ICD ou d'une récidive d'ICD traité par antibiotiques (Gerding, D. N. et al. Administration of Spores of Nontoxigenic Clostridioides difficile Strain M3 for Prevention of Recurrent C difficile Infection: A Randomized Clinical Trial. JAMA 313, 1719 (2015*).*). Les patients ayant reçu la souche M3 après traitement antibiotique ont présenté significativement moins de récidives à 6 semaines que les patients recevant le placebo (12% *vs* 30%). Toutefois, l'utilisation de souches non toxinogènes dans la prévention primaire et/ou le traitement d'un premier épisode d'ICD n'a pas été étudiée.

Il apparait donc nécessaire de mettre au point de nouvelles souches non toxinogènes ainsi que de nouvelles compositions pharmaceutiques permettant de prévenir et de traiter efficacement et durablement un patient atteint d'une infection à *Clostridioides difficile,* tout en limitant les effets secondaires, les risques de récidives et en améliorant le temps de réponse au traitement.

Le but de la présente invention est précisément de proposer de nouvelles souches non toxinogènes ainsi que des compositions pharmaceutiques permettant de prévenir et/ou de traiter efficacement les patients atteints d'infections associées à *Clostridioides difficile.*

**Résumé de l'invention**

La présente invention a donc pour objet une souche non toxinogène de *Clostridioides difficile* pour son utilisation dans la prévention primaire et/ou le traitement des infections associées à *Clostridioides difficile* et/ou des récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847). Avantageusement, la souche non toxinogène de *Clostridioides difficile* est isolée à partir de selles de prématurés âgés de moins de 32 semaines aménorrhées. Dans un mode de réalisation particulièrement avantageux de l'invention, la souche non toxinogène de ribotype CE(032), a été déposée le 12 février 2020 auprès DSMZ (Leibniz Institute Deutsche Sammlung von Mikroorganismen und Zellkulturen GmbH) sous le numéro DSM 33438. Dans un autre mode de réalisation particulièrement avantageux de l'invention, la souche non toxinogène de ribotype CE(847) a été déposée le 12 février 2020 auprès DSMZ (Leibniz Institute Deutsche Sammlung von Mikroorganismen und Zellkulturen GmbH) sous le numéro DSM 33437.

Un autre objet de l'invention porte sur une composition pharmaceutique comprenant au moins une souche non toxinogène de *Clostridioides difficile* comme principe actif, pour son utilisation dans la prévention primaire et/ou le traitement des infections associées à *Clostridioides difficile* et/ou récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847). Dans un mode de réalisation particulièrement avantageux de l'invention, la souche non toxinogène de ribotype CE(032) de la composition pharmaceutique a été déposée le 12 février 2020 auprès DSMZ (Leibniz Institute Deutsche Sammlung von Mikroorganismen und Zellkulturen GmbH) sous le numéro DSM 33438. Dans un autre mode de réalisation particulièrement avantageux de l'invention, la souche non toxinogène de ribotype CE(847) de la composition pharmaceutique a été déposée le 12 février 2020 auprès DSMZ (Leibniz Institute Deutsche Sammlung von Mikroorganismen und Zellkulturen GmbH) sous le numéro DSM 33437.

Dans un mode de réalisation particulier de l'invention, la composition pharmaceutique comprend en outre un ou plusieurs excipients pharmaceutiquement acceptables. Dans un mode de réalisation particulier de l'invention, la composition pharmaceutique comprend une quantité thérapeutiquement efficace de souche non toxinogène de *Clostridioides difficile.* Dans un mode de réalisation particulier de l'invention, la composition pharmaceutique est sous une forme adaptée pour son administration par voie orale et/ou sous forme de compléments alimentaires et/ou produits alimentaires.

**Description détaillée de l'invention**

La présente invention a donc pour objet une souche non toxinogène de *Clostridioides difficile* pour son utilisation dans la prévention primaire et/ou le traitement des infections associées à *Clostridioides difficile* et/ou de ces récidives, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847).

Les inventeurs ont montré de manière surprenante que l'administration de manière préventive d'une souche non toxinogène de ribotype CE(032) et/ou d'une souche non toxinogène de ribotype CE(847) à des hamsters permet de réduire de plus de 50% le risque d'infections fatales associées à *Clostridioides difficile,* en particulier associées à la souche hypervirulente de ribotype PCR 027, par rapport à des hamsters n'ayant pas reçus préalablement l'une des souches non toxinogènes de ribotype CE(032) et/ou de ribotype CE(847). Avantageusement, les inventeurs ont montré que l'administration de manière préventive de la souche non toxinogène de ribotype CE(032) permet de réduire de 89% le risque d'infections fatales associées à *Clostridioides difficile,* en particulier associées à la souche hypervirulente de ribotype PCR 027. Avantageusement, les inventeurs ont montré que l'administration de manière préventive de la souche non toxinogène de ribotype CE(847) permet de réduire de 56% le risque d'infections fatales associées à *Clostridioides difficile,* en particulier associées à la souche hypervirulente de ribotype PCR 027.

De plus, les inventeurs ont montré que les souches non toxinogènes de ribotype CE(032) et de ribotype CE(847) sont respectivement capables d'inhiber la croissance de souche toxinogène, en particulier la croissance de la souche hypervirulente de ribotype PCR 027, lorsque les souches non toxinogènes de ribotype CE(032) et de ribotype CE(847) sont respectivement mises en co-culture en milieu liquide avec la souche hypervirulente de ribotype PCR 027.

Par ailleurs, les inventeurs ont montré que les souches non toxinogènes de ribotype CE(032) et de ribotype CE(847) selon l'invention n'avaient aucun effet néfaste sur le microbiote des patients, auxquels les souches non toxinogènes de ribotype CE(032) et de ribotype CE(847) selon l'invention peuvent être administrées, quel que soit le mode et la voie d'administration. En d'autres termes, les inventeurs ont montré que les souches non toxinogènes de ribotype CE(032) et de ribotype CE(847) selon l'invention n'altèrent pas la flore microbienne existante.

Au sens de la présente invention, le terme «souche non toxinogène» désigne une souche de *Clostridioides difficile* dépourvue de production de toxines A (TcdA) et/ou B (TcdB) ou produisant moins de 5% de toxines A (TcdA) et/ou B (TcdB). Avantageusement, une souche est dite non toxinogène, si la production de toxines A et/ou B est inférieure à 5%, avantageusement inférieure à 4%, avantageusement inférieure à 3%, avantageusement inférieure à 2%, avantageusement inférieure à 1%, avantageusement inférieure à 0,5%, avantageusement inférieure à 0,4%, avantageusement inférieure à 0,3%, avantageusement inférieure à 0,2%, avantageusement inférieure à 0,1%. Dans un mode de réalisation particulièrement avantageux, la souche est une souche non toxinogène dépourvue de production de toxines A et/ou B. Au sens de la présente invention, on entend par « dépourvue de production de toxines A et/ou B », une souche ne produisant pas de toxines A et/ou B. En d'autres termes, une souche dépourvue de production de toxines A et/ou B est une souche dans laquelle la production de toxines A et/ou B est nulle. Dans un mode de réalisation particulièrement avantageux de l'invention, une souche non toxinogène dépourvue de production de toxines A et/ou B est une souche dans laquelle le locus de pathogénicité PaLoc sur lequel sont localisés un ou plusieurs gènes codant pour la production des toxines A et/ou B est absent ou inactivé. Dans un mode de réalisation particulier de l'invention, le locus de pathogénicité peut être soit naturellement absent, soit inactivé par n'importe quelle technique d'inactivation bien connue de l'homme du métier, et notamment par des moyens génétiques, des techniques enzymatiques, des procédés chimiques ou des combinaisons de celles-ci. L'inactivation peut être effectuée au niveau de l'ADN, de l'ARNm ou de la protéine et inhiber l'expression des gènes codant pour la production des toxines A et/ou B ou l'activité des toxines A et/ou B. Avantageusement, les méthodes d'inactivation affectent l'expression des gènes codant pour la production des toxines A et/ou B et conduisent à l'absence de production des toxines A et/ou B.

Au sens de la présente invention, le terme « souche non toxinogène de *Clostridioides difficile* » désigne une souche de *Clostridioides difficile* dépourvue de production de toxines A (TcdA) et/ou B (TcdB). En d'autres termes, la souche non toxinogène de *Clostridioides difficile* selon l'invention ne produit pas de toxine A et/ou de toxine B.

Dans un mode de réalisation particulièrement avantageux, la souche non toxinogène de *Clostridioides difficile* selon l'invention est dépourvue de production de toxine A. En d'autres termes, la souche non toxinogène de *Clostridioides difficile* selon l'invention ne produit pas de toxine A. Avantageusement, la souche non toxinogène de *Clostridioides difficile* selon l'invention ne produit pas naturellement de toxine A. Dans un mode de réalisation particulièrement avantageux, la souche non toxinogène de *Clostridioides difficile* selon l'invention est dépourvue de production de toxine B. En d'autres termes, la souche non toxinogène de *Clostridioides difficile* selon l'invention ne produit pas de toxine B. Avantageusement, la souche non toxinogène de *Clostridioides difficile* selon l'invention ne produit pas naturellement de toxine B. Dans un mode de réalisation particulièrement avantageux, la souche non toxinogène de *Clostridioides difficile* selon l'invention est dépourvue de production de toxines A et B. En d'autres termes, la souche non toxinogène de *Clostridioides difficile* selon l'invention ne produit pas de toxines A et B. Avantageusement, la souche non toxinogène de *Clostridioides difficile* selon l'invention ne produit pas naturellement de toxines A et B.

Dans un mode de réalisation particulièrement avantageux de l'invention, la souche non toxinogène de *Clostridioides difficile* est isolée à partir de selles de nouveau-nés prématurés. Au sens de la présente invention, le terme « isolée » désigne une souche non toxinogène de *Clostridioides difficile* qui a été séparée de l'environnement dans laquelle elle se trouve naturellement, pour exister sous une forme essentiellement pure. Le terme « isolée » ne signifie pas nécessairement l'exclusion des mélanges artificiels ou synthétiques avec d'autres composés ou matériaux, ni la présence d'impuretés n'interférant pas avec l'activité fondamentale et pouvant être présentes, par exemple, en raison d'une purification incomplète. Dans un mode de réalisation particulièrement avantageux de l'invention, la souche non toxinogène de *Clostridioides difficile* est dérivée de selles de nouveau-nés prématurés. Dans un mode de réalisation particulièrement avantageux de l'invention, les nouveau-nés prématurés sont des nouveau-nés prématurés âgés de moins de 36 semaines aménorrhées (SA), avantageusement de moins de 35 semaines aménorrhées, avantageusement de moins de 34 semaines aménorrhées, avantageusement de moins de 33 semaines aménorrhées, avantageusement de moins de 32 semaines aménorrhées, avantageusement de moins de 30 semaines aménorrhées, avantageusement de moins de 29 semaines aménorrhées, avantageusement de moins de 28 semaines aménorrhées.

Selon un mode de réalisation particulier de l'invention, les caractéristiques génétiques des souches non toxinogènes selon l'invention, et en particulier des souches non toxinogènes ont été déterminées par ribotypage. Avantageusement, cette méthode de typage explore le polymorphisme des régions intergéniques (ou entretoises) situées entre l'ADN codant pour l'ARN 16S et 23S. Il s'agit de la méthode européenne de référence de typage des souches de *Clostridioides difficile* qui a fait l'objet d'une standardisation par l'ECDC (https://www.ecdc.europa.eu/en/publications-data/laboratory-procedures-diagnosis-and-typing-human-clostridium-difficile-infection).

Dans un mode de réalisation particulièrement avantageux de l'invention, la souche non toxinogène de *Clostridioides difficile* est la souche non toxinogène de ribotype CE(032), déposée le 12 février 2020 auprès du DSMZ (Leibniz Institute Deutsche Sammlung von Mikroorganismen und Zellkulturen GmbH) sous le numéro DSM 33438.

Dans un autre mode de réalisation particulièrement avantageux de l'invention, la souche non toxinogène de *Clostridioides difficile* est la souche non toxinogène de ribotype CE(847), déposée le 12 février 2020 auprès du DSMZ (Leibniz Institute Deutsche Sammlung von Mikroorganismen und Zellkulturen GmbH) sous le numéro DSM 33437.

Un autre aspect de l'invention concerne une composition pharmaceutique comprenant au moins une souche non toxinogène de *Clostridioides difficile* comme principe actif, pour son utilisation dans la prévention primaire et/ou le traitement des infections associées à *Clostridioides difficile* et/ou de les récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847).

Au sens de la présente invention, on entend par « composition pharmaceutique » aussi bien une composition pharmaceutique sous forme liquide, en particulier sous forme de solution ou de suspension qu'une composition pharmaceutique sous forme de poudre.

Dans un mode de réalisation particulièrement avantageux de l'invention, la souche non toxinogène de *Clostridioides difficile* est utilisée comme seul principe actif. En d'autres termes, la composition pharmaceutique selon l'invention ne comprend qu'une souche non toxinogène de *Clostridioides difficile* comme unique principe actif. On entend par « principe actif », un composé ayant une activité biologique capable de prévenir et/ou de traiter les infections à *Clostridioides difficile* et/ou les récidives associées.

Dans un mode de réalisation particulièrement avantageux de l'invention, la souche non toxinogène de la composition pharmaceutique est la souche non toxinogène de ribotype CE(032), déposée le 12 février 2020 auprès du DSMZ (Leibniz Institute Deutsche Sammlung von Mikroorganismen und Zellkulturen GmbH) sous le numéro DSM 33438.

Dans un autre mode de réalisation particulièrement avantageux de l'invention, la souche non toxinogène de la composition pharmaceutique est la souche non toxinogène de ribotype CE(847), déposée le 12 février 2020 auprès du DSMZ (Leibniz Institute Deutsche Sammlung von Mikroorganismen und Zellkulturen GmbH) sous le numéro DSM 33437.

Dans un mode de réalisation particulièrement avantageux de l'invention, la souche non toxinogène de ribotype CE(032) est utilisée comme seul principe actif. En d'autres termes, la composition pharmaceutique selon l'invention ne comprend qu'une souche non toxinogène de ribotype CE(032) comme unique principe actif.

Dans un autre mode de réalisation particulièrement avantageux de l'invention, la souche non toxinogène de ribotype CE(847) est utilisée comme seul principe actif. En d'autres termes, la composition pharmaceutique selon l'invention ne comprend qu'une souche non toxinogène de ribotype CE(847) comme unique principe actif.

Dans un autre mode de réalisation particulièrement avantageux de l'invention, le mélange des souches non toxinogènes de ribotype CE(032) et de ribotype CE(847) est utilisé comme seul principe actif. En d'autres termes, la composition pharmaceutique selon l'invention ne comprend que le mélange des souches non toxinogènes de ribotype CE(032) et de ribotype CE(847) comme unique principe actif. Avantageusement, lorsque les souches non toxinogènes de ribotype CE(032) et de ribotype CE(847) sont utilisées sous la forme d'un mélange, la répartition (également appelé le ratio ou la proportion) entre la souche non toxinogène de ribotype CE(032) et la souche non toxinogène de ribotype CE(847) dans le mélange est comprise entre 1 :100 et 100:1. Avantageusement, la répartition entre la souche non toxinogène de ribotype CE(032) et la souche non toxinogène de ribotype CE(847) dans le mélange est comprise entre 5 :95 et 95 :5, avantageusement 10 :90 et 90 :10, avantageusement entre 15 :85 et 85 :15, avantageusement entre 20 :80 et 80 :20, avantageusement 25 :75 et 75 :25, avantageusement 30 :70 et 70 :30, avantageusement 35 :65 et 65 :35, avantageusement entre 40 :60 et 60 :40, avantageusement entre 45 :55 et 55 :45. Dans un mode de réalisation particulièrement avantageux, la répartition entre la souche non toxinogène de ribotype CE(032) et la souche non toxinogène de ribotype CE(847) dans le mélange est de 50 :50.

Dans un mode de réalisation particulier de l'invention, la composition pharmaceutique comprend une quantité thérapeutiquement efficace de souche non toxinogène de *Clostridioides difficile.* Avantageusement, la composition pharmaceutique comprend une quantité thérapeutiquement efficace de souche non toxinogène de ribotype CE(032). Avantageusement, la composition pharmaceutique comprend une quantité thérapeutiquement efficace de souche non toxinogène de ribotype CE(847). Avantageusement, la composition pharmaceutique comprend une quantité thérapeutiquement efficace du mélange des souches non toxinogènes de ribotype CE(032) et de ribotype CE(847).

Selon un mode de réalisation avantageux de l'invention, on entend par « une quantité thérapeutiquement efficace » ou « une quantité efficace pour traiter » ou « une quantité efficace sur le plan pharmaceutique » de la souche non toxinogène de *Clostridioides difficile* selon l'invention, la quantité de souche non toxinogène de *Clostridioides difficile* ou d'une composition comprenant la souche non toxinogène de *Clostridioides difficile* nécessaire pour inhiber ou inverser une maladie (par exemple, pour traiter les infections à *Clostridioides difficile*). La détermination clinique d'une quantité thérapeutiquement efficace dépend spécifiquement de facteurs tels que l'absence de toxicité et l'efficacité du médicament. Ces facteurs différeront en fonction d'autres facteurs tels que la puissance, la biodisponibilité relative, le poids corporel du patient, la gravité des effets indésirables et le mode d'administration préféré. La toxicité peut être déterminée en utilisant des procédés bien connus dans la technique. L'efficacité peut être déterminée en utilisant les mêmes indications. L'efficacité peut être mesurée en mesurant le taux de survie des patients traités. Une quantité pharmaceutiquement efficace est donc une quantité que le clinicien considère comme tolérable sur le plan toxicologique, mais efficace, c'est-à-dire que la quantité administrée confère au moins une amélioration clinique de l'état du patient.

Le dosage peut être ajusté de manière appropriée afin de garantir une colonisation rapide et efficace du tractus gastro-intestinal par la souche non toxinogène. Au sens de la présente invention, on entend par « tractus gastro-intestinal » ou « tube digestif », l'ensemble des organes creux constituant le trajet des aliments depuis la cavité buccale jusqu'au rectum, ce qui comprend la cavité buccale, l'œsophage, l'estomac, le duodénum, le jéjunum, l'iléon, le caecum, le côlon ascendant, le côlon transverse, le côlon descendant et sigmoïde, le rectum. Avantageusement, la souche non toxinogène de *Clostridioides difficile* selon l'invention est particulièrement efficace pour coloniser le duodénum, le jéjunum, l'iléon, le caecum, le côlon ascendant, le côlon transverse et le côlon descendant et sigmoïde.

Dans un mode de réalisation particulièrement avantageux selon l'invention, la souche non toxinogène de *Clostridioides difficile* selon l'invention est capable de coloniser l'intestin en moins de 96 heures après son administration. Avantageusement, la souche non toxinogène de *Clostridioides difficile* selon l'invention est capable de coloniser l'intestin en moins de 72 heures, avantageusement en moins de 48 heures, avantageusement en moins de 24 heures, avantageusement en moins de 12 heures, avantageusement en moins de 6 heures après son administration. Avantageusement, la souche non toxinogène de *Clostridioides difficile* selon l'invention est capable de coloniser l'intestin dans un intervalle de temps compris entre 6 heures et 96 heures après son administration, avantageusement dans un intervalle de temps compris entre 12 heures et 72 heures après son administration, avantageusement dans un intervalle de temps compris entre 24 heures et 72 heures après son administration, avantageusement dans un intervalle de temps compris entre 24 heures et 48 heures après son administration. Dans un mode de réalisation particulièrement avantageux selon l'invention, la souche non toxinogène de *Clostridioides difficile* selon l'invention est capable de coloniser l'intestin en moins de 48 heures après son administration.

Dans le cas où la réponse chez le patient est insuffisante à de telles doses, des doses encore plus élevées (ou des doses efficaces plus élevées par une voie d'administration différente et plus localisée) peuvent être utilisées dans la mesure où la tolérance du patient le permet. Des doses multiples par jour peuvent également être utilisées. « Dose », « quantité » et « dosage » sont utilisés ici de manière interchangeable. Dans un mode de réalisation particulièrement avantageux de l'invention, la souche non toxinogène de *Clostridioides difficile* selon l'invention peut être administrée à des intervalles et à des doses appropriés pour établir d'abord une colonisation du tractus gastro-intestinal, puis la posologie peut être réduite à un niveau de maintien, afin de maintenir la colonisation et l'élimination des spores.

Dans un mode de réalisation particulièrement avantageux de l'invention, la quantité de souche non toxinogène de *Clostridioides difficile* selon l'invention ou de composition pharmaceutique comprenant au moins une souche non toxinogène de *Clostridioides difficile* en tant que seul principe actif administrée au patient est d'au moins une spore, avantageusement au moins 10 spores, avantageusement au moins 50 spores, avantageusement au moins $10^2$ spores, avantageusement au moins $10^3$ spores, avantageusement au moins $10^4$ spores, avantageusement au moins $10^5$ spores, avantageusement au moins $10^6$ spores, avantageusement au moins $10^7$ spores, avantageusement au moins $10^8$ spores, avantageusement au moins $10^9$ spores, avantageusement au moins $10^{10}$ spores, avantageusement au moins $10^{11}$ spores, avantageusement au moins $10^{12}$ spores, ou plus en une ou plusieurs doses. Avantageusement, la quantité de souche non toxinogène de *Clostridioides difficile* selon l'invention ou de composition pharmaceutique comprenant au moins une souche non toxinogène de *Clostridioides difficile* en tant que seul principe actif administrée au patient est comprise entre une spore et $10^{12}$ spores, avantageusement comprise entre 10 spores et $10^{11}$ spores, avantageusement entre 50 spores et $10^{10}$ spores, avantageusement entre $10^2$ spores et $10^9$ spores, avantageusement entre $10^3$ spores et $10^8$ spores, avantageusement entre $10^4$ spores et $10^7$ spores, en une ou plusieurs doses.

Les doses peuvent être administrées à raison d'une fois par jour ou plus d'une fois par jour, et pendant plusieurs jours. Avantageusement, les doses peuvent être administrées pendant un jour, avantageusement pendant 2 jours, avantageusement pendant 3 jours, avantageusement pendant 4 jours, avantageusement pendant 5 jours, avantageusement pendant 6 jours, avantageusement pendant 7 jours, avantageusement pendant 8 jours, avantageusement pendant 9 jours, avantageusement pendant 10 jours, avantageusement pendant 11 jours, avantageusement pendant 12 jours, avantageusement pendant 13 jours, avantageusement pendant 14 jours, avantageusement pendant 15 jours, avantageusement pendant 16 jours, avantageusement pendant 17 jours, avantageusement pendant 18 jours avantageusement pendant 19 jours, avantageusement pendant 20 jours, avantageusement pendant 21 jours, avantageusement pendant 22 jours, avantageusement pendant 23 jours, avantageusement pendant 24 jours, avantageusement pendant 25 jours, avantageusement pendant 26 jours, avantageusement pendant 27 jours, avantageusement pendant 28 jours, avantageusement pendant 29 jours, avantageusement pendant 30 jours, avantageusement pendant 31 jours, avantageusement pendant un mois.

La souche non toxinogène de *Clostridioides difficile* selon l'invention peut être administrée à des intervalles et à des doses appropriées pour établir d'abord une colonisation du tractus gastro-intestinal, après quoi la posologie peut être réduite à un niveau de maintien afin de maintenir la colonisation et l'élimination des spores.

Dans un mode de réalisation particulièrement avantageux de l'invention, la composition pharmaceutique comprenant au moins une souche non toxinogène de *Clostridioides difficile* comme principe actif, comprend en outre au moins un excipient pharmaceutiquement acceptable.

Dans un mode de réalisation avantageux de l'invention, l'au moins un excipient pharmaceutique acceptable est choisi parmi les excipients suivants : l'acide caprylique ou un sel de caprylate, le glucose, le fructose, le rhamnose, le galactose, le mannose, le sorbose, le saccharose, le ribose, le désoxyribose, le sucrose, le maltitol, l'isomaltitol, le lactitol, l'inuline, la lécithine, le tréhalose, le lactose, le maltose, le raffinose, le mannitol, le sorbitol, le glycérol, l'arabitol, le lactitol, de xylitol, la ribitol, le dulcitol, le volemitol, le polypropylène glycol, le polyéthylène glycol, l'ester d'acides gras de sorbitane polyoxyéthylène, le polysorbate 20, le polysorbate 80, les poloxamers, les éthers d'alkyles de polyoxyéthylène, les éthers d'alkylphènylpolyoxyéthylène, le copolymère de polyoxyéthylène-polyoxypropylène, le dodécylsulphate de sodium, le succinate de sodium, le chlorure de sodium, le citrate de sodium, l'acide citrique monohydraté, le phosphate de sodium dibasique dihydraté, le phosphate de sodium monobasique dihydraté, le phosphate de sodium monobasique monohydraté ou d'EDTA, les cyclodextrines, le chitosan et le carboxyméthyle-dextran, la liste n'étant pas limitative.

Dans un mode de réalisation avantageux de l'invention, la cyclodextrine peut être choisie parmi l'a-cyclodextrine, la β-cyclodextrine, la γ-cyclodextrine, un de leurs dérivés, comme notamment un dérivé hydroxypropyle, hydroxyéthyle, éthyle ou méthyle, une cyclodextrine béta éther sulfobutyle, une cyclodextrine branchée, un polymère à base de cyclodextrine ou un mélange de ceux-ci.

Avantageusement, la composition pharmaceutique selon l'invention peut comprendre une combinaison d'excipients pharmaceutiquement acceptables. Dans un mode de réalisation particulier de l'invention, la composition pharmaceutique selon l'invention peut comprendre au moins deux excipients pharmaceutiquement acceptables différents, avantageusement au moins trois excipients pharmaceutiquement acceptables différents, avantageusement au moins quatre excipients pharmaceutiquement acceptables différents, avantageusement au moins cinq excipients pharmaceutiquement acceptables différents, avantageusement au moins six excipients pharmaceutiquement acceptables différents, ou plus.

Dans un mode de réalisation particulièrement avantageux de l'invention, la composition pharmaceutique comprenant au moins une souche non toxinogène de *Clostridioides difficile* comme principe actif, comprend au moins une spore, avantageusement au moins 10 spores, avantageusement au moins 50 spores, avantageusement au moins $10^2$ spores, avantageusement au moins $10^3$ spores, avantageusement au moins $10^4$ spores, avantageusement au moins $10^5$ spores, avantageusement au moins $10^6$ spores, avantageusement au moins $10^7$ spores, avantageusement au moins $10^8$ spores, avantageusement au moins $10^9$ spores, avantageusement au moins $10^{10}$ spores, avantageusement au moins $10^{11}$ spores, avantageusement au moins $10^{12}$ spores, ou plus. Avantageusement, la composition pharmaceutique comprenant au moins une souche non toxinogène de *Clostridioides difficile* comme principe actif, comprend entre une spore et $10^{12}$ spores, avantageusement comprise entre 10 spores et $10^{11}$ spores, avantageusement entre 50 spores et $10^{10}$ spores, avantageusement entre $10^2$ spores et $10^9$ spores, avantageusement entre $10^3$ spores et $10^8$ spores, avantageusement entre $10^4$ spores et $10^8$ spores, avantageusement entre $10^4$ spores et $10^7$ spores.

Dans un mode de réalisation particulièrement avantageux de l'invention, la composition pharmaceutique comprenant au moins une souche non toxinogène de ribotype CE(032) comme principe actif, comprend au moins une spore, avantageusement au moins 10 spores, avantageusement au moins 50 spores, avantageusement au moins $10^2$ spores, avantageusement au moins $10^3$ spores, avantageusement au moins $10^4$ spores, avantageusement au moins $10^5$ spores, avantageusement au moins $10^6$ spores, avantageusement au moins $10^7$ spores, avantageusement au moins $10^8$ spores, avantageusement au moins $10^9$ spores, avantageusement au moins $10^{10}$ spores, avantageusement au moins $10^{11}$ spores, avantageusement au moins $10^{12}$ spores, ou plus. Avantageusement, la composition pharmaceutique comprenant au moins une souche non toxinogène de ribotype CE(032) comme principe actif, comprend entre une spore et $10^{12}$ spores, avantageusement comprise entre 10 spores et $10^{11}$ spores, avantageusement entre 50 spores et $10^{10}$ spores, avantageusement entre $10^2$ spores et $10^9$ spores, avantageusement entre $10^3$ spores et $10^8$ spores, avantageusement entre $10^4$ spores et $10^8$ spores, avantageusement entre $10^4$ spores et $10^7$ spores.

Dans un mode de réalisation particulièrement avantageux de l'invention, la composition pharmaceutique comprenant au moins une souche non toxinogène de ribotype CE(847) comme principe actif, comprend au moins une spore, avantageusement au moins 10 spores, avantageusement au moins 50 spores, avantageusement au moins $10^2$ spores, avantageusement au moins $10^3$ spores, avantageusement au moins $10^4$ spores, avantageusement au moins $10^5$ spores, avantageusement au moins $10^6$ spores, avantageusement au moins $10^7$ spores, avantageusement au moins $10^8$ spores, avantageusement au moins $10^9$ spores, avantageusement au moins $10^{10}$ spores, avantageusement au moins $10^{11}$ spores, avantageusement au moins $10^{12}$ spores, ou plus. Avantageusement, la composition pharmaceutique comprenant au moins une souche non toxinogène de ribotype CE(847) comme principe actif, comprend entre une spore et $10^{12}$ spores, avantageusement comprise entre 10 spores et $10^{11}$ spores, avantageusement entre 50 spores et $10^{10}$ spores, avantageusement entre $10^2$ spores et $10^9$ spores, avantageusement entre $10^3$ spores et $10^8$ spores, avanta-

geusement entre $10^4$ spores et $10^8$ spores, avantageusement entre $10^4$ spores et $10^7$ spores.

Dans un mode de réalisation particulièrement avantageux de l'invention, la composition pharmaceutique comprenant au moins un mélange de souche non toxinogène de ribotype CE(032) et une souche non toxinogène de ribotype CE(847) comme principe actif, comprend au moins une spore, avantageusement au moins 10 spores, avantageusement au moins 50 spores, avantageusement au moins $10^2$ spores, avantageusement au moins $10^3$ spores, avantageusement au moins $10^4$ spores, avantageusement au moins $10^5$ spores, avantageusement au moins $10^6$ spores, avantageusement au moins $10^7$ spores, avantageusement au moins $10^8$ spores, avantageusement au moins $10^9$ spores, avantageusement au moins $10^{10}$ spores, avantageusement au moins $10^{11}$ spores, avantageusement au moins $10^{12}$ spores, ou plus. Avantageusement, la composition pharmaceutique comprenant au moins un mélange de souche non toxinogène de ribotype CE(032) et une souche non toxinogène de ribotype CE(847) comme principe actif, comprend entre une spore et $10^{12}$ spores, avantageusement comprise entre 10 spores et $10^{11}$ spores, avantageusement entre 50 spores et $10^{10}$ spores, avantageusement entre $10^2$ spores et $10^9$ spores, avantageusement entre $10^3$ spores et $10^8$ spores, avantageusement entre $10^4$ spores et $10^8$ spores, avantageusement entre $10^4$ spores et $10^7$ spores.

Dans un mode de réalisation particulièrement avantageux de l'invention, la composition pharmaceutique comprenant la souche non toxinogène de *Clostridioides difficile* comme principe actif, comprend au moins $10^4$ spores d'une souche non toxinogène de *Clostridioides difficile* comme principe actif.

Avantageusement, la composition pharmaceutique comprend au moins $10^4$ spores d'une souche non toxinogène de ribotype CE(032) selon l'invention comme principe actif. Avantageusement, la composition pharmaceutique comprend au moins $10^5$ spores d'une souche non toxinogène de ribotype CE(032) selon l'invention comme principe actif. Avantageusement, la composition pharmaceutique comprend au moins $10^6$ spores d'une souche non toxinogène de ribotype CE(032) selon l'invention comme principe actif. Avantageusement, la composition pharmaceutique comprend au moins $10^7$ spores d'une souche non toxinogène de ribotype CE(032) selon l'invention comme principe actif. Avantageusement, la composition pharmaceutique comprend au moins $10^8$ spores d'une souche non toxinogène de ribotype CE(032) selon l'invention comme principe actif. Avantageusement, la composition pharmaceutique comprend au moins $10^9$ spores d'une souche non toxinogène de ribotype CE(032) selon l'invention comme principe actif. Avantageusement, la composition pharmaceutique comprend au moins $10^{10}$ spores d'une souche non toxinogène de ribotype CE(032) selon l'invention comme principe actif. Avantageusement, la composition pharmaceutique comprend au moins $10^{11}$ spores d'une souche non toxinogène de ribotype CE(032) selon l'invention comme principe actif. Avantageusement, la composition pharmaceutique comprend au moins $10^{12}$ spores d'une souche non toxinogène de ribotype CE(032) selon l'invention comme principe actif.

Avantageusement, la composition pharmaceutique comprend au moins $10^4$ spores d'une souche non toxinogène de ribotype CE(847) selon l'invention comme principe actif. Avantageusement, la composition pharmaceutique comprend au moins $10^5$ spores d'une souche non toxinogène de ribotype CE(847) selon l'invention comme principe actif. Avantageusement, la composition pharmaceutique comprend au moins $10^6$ spores d'une souche non toxinogène de ribotype CE(847) selon l'invention comme principe actif. Avantageusement, la composition pharmaceutique comprend au moins $10^7$ spores d'une souche non toxinogène de ribotype CE(847) selon l'invention comme principe actif. Avantageusement, la composition pharmaceutique comprend au moins $10^8$ spores d'une souche non toxinogène de ribotype CE(847) selon l'invention comme principe actif. Avantageusement, la composition pharmaceutique comprend au moins $10^9$ spores d'une souche non toxinogène de ribotype CE(847) selon l'invention comme principe actif. Avantageusement, la composition pharmaceutique comprend au moins $10^{10}$ spores d'une souche non toxinogène de ribotype CE(847) selon l'invention comme principe actif. Avantageusement, la composition pharmaceutique comprend au moins $10^{11}$ spores d'une souche non toxinogène de ribotype CE(847) selon l'invention comme principe actif. Avantageusement, la composition pharmaceutique comprend au moins $10^{12}$ spores d'une souche non toxinogène de ribotype CE(847) selon l'invention comme principe actif.

Avantageusement, la composition pharmaceutique comprend au moins $10^4$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^5$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^6$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^7$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^8$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^9$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^{10}$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^{11}$ spores d'un mélange d'une souche non toxinogène de ribotype CE(032) et d'une souche non toxinogène de ribotype CE(847) selon l'invention comme principe actif. Avantageusement, la composition pharmaceutique comprend au moins $10^{12}$ spores d'un mélange d'une souche non toxinogène de ribotype CE(032) et d'une souche non toxinogène de ribotype CE(847) selon l'invention comme principe actif.

Avantageusement, lorsque les souches non toxinogènes de ribotype CE(032) et de ribotype CE(847) sont utilisées sous la forme d'un mélange, la répartition (également appelé le ratio ou la proportion) entre la souche non toxinogènes de ribotype CE(032) et la souche non toxinogènes de ribotype CE(847) dans le mélange est comprise entre 1 :100 et 100:1. Avantageusement, la répartition entre la souche non toxinogène de ribotype CE(032) et la souche non toxinogène de ribotype CE(847) dans le mélange est comprise entre 5 :95 et 95:5, avantageusement 10:90 et 90:10, avantageusement entre 15:85 et 85:15, avantageusement entre 20:80 et 80:20, avantageusement 25:75 et 75:25, avantageusement 30 :70 et 70 :30, avantageusement 35 :65 et 65 :35, avantageusement entre 40 :60 et 60 : 40, avantageusement entre 45 :55 et 55 :45. Dans un mode de réalisation particulièrement avantageux, la répartition entre la souche non toxinogène de ribotype CE(032) et la souche non toxinogène de ribotype CE(847) dans le mélange est de 50 :50.

Dans un mode de réalisation particulièrement avantageux de l'invention, la composition pharmaceutique peut être administrée à raison d'une fois par jour ou plus d'une fois par jour, et pendant plusieurs jours. Avantageusement, la composition pharmaceutique peut être administrée pendant un jour, avantageusement pendant 2 jours, avantageusement pendant 3 jours, avantageusement pendant 4 jours, avantageusement pendant 5 jours, avantageusement pendant 6 jours, avantageusement pendant 7 jours, avantageusement pendant 8 jours, avantageusement pendant 9 jours, avantageusement pendant 10 jours, avantageusement pendant 11 jours, avantageusement pendant 12 jours, avantageusement pendant 13 jours, avantageusement pendant 14 jours, avantageusement pendant 15 jours, avantageusement pendant 16 jours, avantageusement pendant 17 jours, avantageusement pendant 18 jours, avantageusement pendant 19 jours, avantageusement pendant 20 jours, avantageusement pendant 21 jours, avantageusement pendant 22 jours, avantageusement pendant 23 jours, avantageusement pendant 24 jours, avantageusement pendant 25 jours, avantageusement pendant 26 jours, avantageusement pendant 27 jours, avantageusement pendant 28 jours, avantageusement pendant 29 jours, avantageusement pendant 30 jours, avantageusement pendant 31 jours, avantageusement pendant un mois.

Dans un mode de réalisation avantageux de l'invention, la composition pharmaceutique selon l'invention, est administrée à une quantité d'au moins $10^4$ spores d'une souche non toxinogène de *Clostridioides difficile* comme principe actif à raison d'une administration par jour.

Dans un mode de réalisation avantageux de l'invention, la composition pharmaceutique selon l'invention, c'est-à-dire la composition pharmaceutique comprenant au moins une souche non toxinogène de *Clostridioides difficile* comme seul principe actif telle que définie précédemment est utilisée pour des applications *in vivo.* En fonction du mode d'administration prévu *in vivo,* la composition pharmaceutique utilisée, selon l'invention, peut être sous la forme posologique solide, semi-solide ou liquide telle que, par exemple, comprimés, granules, pilules, poudres, capsules, gels, liquides, suspensions, des sirops. La composition pharmaceutique selon l'invention peut également comprendre, en fonction de la formulation souhaitée, au moins un support ou diluant pharmaceutiquement acceptable, qui sont définis comme des véhicules à base aqueuse couramment utilisés pour formuler des compositions pharmaceutiques pour une administration humaine ou animale. Le diluant est choisi de manière à ne pas affecter l'activité biologique de la souche non toxinogène de *Clostridioides difficile* selon l'invention. Des exemples de tels diluants sont l'eau distillée, le sérum physiologique, la solution de Ringer, la solution de dextrose et la solution de Hank. De plus, la composition pharmaceutique peut également comprendre d'autres supports, adjuvants, stabilisants non toxiques, non thérapeutiques, non immunogènes, etc. Des quantités efficaces d'un tel diluant ou véhicule sont des quantités efficaces pour obtenir une solution pharmaceutiquement acceptable en termes de formulation, solubilité des composants, activité biologique, etc. Dans certains modes de réalisation, les compositions pharmaceutiques fournies ici sont stériles.

L'administration de composition pharmaceutique comprenant au moins une souche non toxinogène de *Clostridioides difficile* comme seul principe actif telle que définie précédemment peuvent se faire par n'importe quelle voie, et notamment par voie orale ou par voie endoscopique. L'homme du métier reconnaît que la voie d'administration varie en fonction du trouble à traiter. Avantageusement, la composition pharmaceutique est administrée sous des formes posologiques unitaires appropriées pour une administration unique de doses précises.

Dans un mode de réalisation particulièrement avantageux de l'invention, la composition pharmaceutique comprenant au moins une souche non toxinogène de *Clostridioides difficile* comme seul principe actif, est sous une forme adaptée pour son administration par voie orale et/ou sous forme de compléments alimentaires et/ou produits alimentaires.

Dans un mode de réalisation particulièrement avantageux, les compositions pharmaceutiques selon l'invention comprenant au moins une souche non toxinogène de *Clostridioides difficile* comme seul principe actif telles que définies précédemment peuvent être sous forme solide. Les compositions pharmaceutiques sous forme solide peuvent être obtenues par toute technique bien connue de l'homme du métier. Avantageusement, les compositions pharmaceutiques sous forme solide peuvent être obtenues par lyophilisation, atomisation de séchage ou par séchage par vaporisation. Les compositions de la présente invention peuvent se présenter sous toutes les formes galéniques normalement utilisées pour une administration orale, notamment sous forme de comprimés, de gélules, de capsules, des tablettes, de poudre, de sirop ou de toute forme pour préparation orale solide ou sous toute forme de préparation buvable. Les compositions selon l'invention peuvent également se présenter sous la forme de compléments alimentaires. Leur formulation est réalisée par les procédés usuels pour produire des comprimés, des gélules, des capsules, des dragées, des émulsions, des gels, des sirops. En particulier, l'au moins une souche non toxinogène de *Clostridioides difficile* selon l'invention peut être incorporée dans toutes formes de compléments alimentaires ou d'aliments enrichis, par exemple, des barres alimentaires, des poudres compactées ou non, des boissons. Les poudres peuvent être diluées dans de l'eau ou être incorporées dans des barres alimentaires. Avantageusement, la composition pharmaceutique comprenant au moins une souche non toxinogène de *Clostridioides difficile* comme seul principe actif, est sous une forme adaptée pour son administration sous forme de produits alimentaires. Par produit alimentaire, on entend tout ce qui est propre à servir d'aliment. L'au moins une souche non toxinogène de *Clostridioides difficile* peut être utilisée comme principe actif soit dans des produits alimentaires en combinaison avec des supports alimentaires de nature protéique, glucidique ou lipidique, soit dans des produits alimentaires destinés à une alimentation particulière. Les conditions opératoires pour préparer ces compositions selon l'invention font partie des connaissances générales de l'homme du métier.

Dans un mode de réalisation, le patient peut être un être humain, homme ou femme ou enfant ou nourrisson, quel que soit l'âge. Dans un autre mode de réalisation, le patient peut être un animal non humain, avantageusement un mammifère non humain, par exemple un chien, un chat, une souris, un rat, un hamster, un lapin, un chinchilla, un cheval, une vache, un porc, un mouton, une chèvre ou un primate.

Un autre aspect de l'invention concerne une souche non toxinogène de *Clostridioides difficile* selon l'invention ou une composition pharmaceutique comprenant au moins une souche non toxinogène de *Clostridioides difficile* comme seul principe actif destinée à être utilisée dans la prévention des infections associées à *Clostridioides difficile* et/ou les récidives associées.

Dans un mode de réalisation particulièrement avantageux de l'invention, la souche non toxinogène de *Clostridioides difficile* selon l'invention ou les compositions pharmaceutiques comprenant au moins une souche non toxinogène de *Clostridioides difficile* comme seul principe actif telles que définies précédemment sont particulièrement utiles pour prévenir ou réduire les infections associées à *Clostridioides difficile* et/ou les récidives associées chez un patient.

Le terme « prévention » ou « prophylaxie » ou « traitement préventif » ou « traitement prophylactique » comprend aussi bien un traitement aboutissant à la prévention d'une maladie qu'un traitement réduisant et/ou retardant l'incidence d'une maladie ou le risque de survenue de la maladie chez un patient.

L'administration d'une souche non toxinogène de *Clostridioides difficile* selon l'invention ou d'une composition comprenant au moins une souche non toxinogène de *Clostridioides difficile* comme seul principe actif, de manière préventive, permet de réduire considérablement les risques d'apparition ou de récidives d'infections associées à *Clostridioides difficile* chez un patient.

Dans un mode de réalisation particulièrement avantageux de l'invention, la souche non toxinogène de *Clostridioides difficile* selon l'invention ou d'une composition comprenant au moins une souche non toxinogène de *Clostridioides difficile* comme seul principe actif, peut être administrée, de manière préventive à raison d'une fois par jour ou plus d'une fois par jour, et pendant plusieurs jours.

Dans un mode de réalisation particulièrement avantageux de l'invention, la souche non toxinogène de *Clostridioides difficile* selon l'invention ou les compositions comprenant au moins une souche non toxinogène de *Clostridioides difficile* comme seul principe actif, peuvent être administrées, sous la forme d'une cure préventive.

Au sens de la présente invention, on entend par « cure préventive », l'administration à un patient en nécessitant, à raison d'une fois par jour pendant 7 jours de la souche non toxinogène de *Clostridioides difficile* selon l'invention ou d'une composition comprenant au moins une souche non toxinogène de *Clostridioides difficile* comme seul principe actif, et cela de manière répétée, avantageusement tous les deux mois, avantageusement tous les trois mois, avantageusement tous les quatre mois, avantageusement tous les cinq mois, avantageusement tous les six mois.

Avantageusement, la souche non toxinogène de *Clostridioides difficile* selon l'invention ou les compositions comprenant au moins une souche non toxinogène de *Clostridioides difficile* comme seul principe actif, peuvent être administrées, de manière répétée à raison d'une fois par jour pendant 7 jours, tous les deux mois.

Avantageusement, la souche non toxinogène de *Clostridioides difficile* selon l'invention ou les compositions comprenant au moins une souche non toxinogène de *Clostridioides difficile* comme seul principe actif, peuvent être administrées, de manière répétée à raison d'une fois par jour pendant 7 jours, tous les trois mois.

Avantageusement, la souche non toxinogène de *Clostridioides difficile* selon l'invention ou les compositions comprenant au moins une souche non toxinogène de *Clostridioides difficile* comme seul principe actif, peuvent être administrées, de manière répétée à raison d'une fois par jour pendant 7 jours, tous les quatre mois.

Avantageusement, la souche non toxinogène de *Clostridioides difficile* selon l'invention ou les compositions comprenant au moins une souche non toxinogène de *Clostridioides difficile* comme seul principe actif, peuvent être administrées, de manière répétée à raison d'une fois par jour pendant 7 jours, tous les cinq mois.

Avantageusement, la souche non toxinogène de *Clostridioides difficile* selon l'invention ou les compositions comprenant au moins une souche non toxinogène de *Clostridioides difficile* comme seul principe actif, peuvent être administrées, de manière répétée à raison d'une fois par jour pendant 7 jours, tous les six mois.

De préférence, les risques d'infections fatales associées à *Clostridioides difficile* sont réduits de 50%. Avantageusement, cette administration de manière préventive d'une souche non toxinogène de *Clostridioides difficile* selon l'invention ou d'une composition comprenant au moins une souche non toxinogène de *Clostridioides difficile* selon l'invention comme seul principe actif permet de réduire de plus de 50% le risque d'infections fatales associées à *Clostridioides difficile,* en particulier associées à la souche hypervirulente de ribotype PCR 027, par rapport à des patients n'ayant pas reçu préalablement au moins l'une des souches non toxinogènes selon l'invention.

Avantageusement, l'administration de manière préventive d'une souche non toxinogène de *Clostridioides difficile* selon l'invention ou d'une composition comprenant au moins une souche non toxinogène de *Clostridioides difficile* selon l'invention comme seul principe actif permet de réduire de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99%, le risque d'infections fatales associées à *Clostridioides difficile,* en particulier associées à la souche hypervirulente de ribotype PCR 027, par rapport à des patients n'ayant pas reçu préalablement au moins l'une des souches non toxinogènes selon l'invention. Dans un mode de réalisation encore plus avantageux, l'administration de manière préventive d'une souche non toxinogène de *Clostridioides difficile* selon l'invention ou d'une composition comprenant au moins une souche non toxinogène de *Clostridioides difficile* selon l'invention comme seul principe actif permet de réduire de 100% le risque d'infections fatales associées à *Clostridioides difficile,* en particulier associées à la souche hypervirulente de ribotype PCR 027, par rapport à des patients n'ayant pas reçu préalablement au moins l'une des souches non toxinogènes selon l'invention.

Dans un mode de réalisation particulièrement avantageux de l'invention, l'administration de manière préventive de la souche non toxinogène de ribotype CE(032) selon l'invention ou d'une composition comprenant au moins une souche non toxinogène de ribotype CE(032) selon l'invention comme seul principe actif permet de réduire de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque d'infections fatales associées à *Clostridioides difficile,* en particulier associées à la souche hypervirulente de ribotype PCR 027, par rapport à des patients n'ayant pas reçu préalablement la souche non toxinogène de ribotype CE(032) selon l'invention. Dans un mode de réalisation encore plus avantageux, l'administration de manière préventive de la souche non toxinogène de ribotype CE(032) selon l'invention ou d'une composition comprenant au moins une souche non toxinogène de ribotype CE(032) selon l'invention comme seul principe actif comme seul principe actif permet de réduire de 100% le risque d'infections fatales associées à *Clostridioides difficile,* en particulier associées à la souche hypervirulente de ribotype PCR 027, par rapport à des patients n'ayant pas reçu préalablement la souche non toxinogène de ribotype CE(032) selon l'invention.

Avantageusement, l'administration de manière préventive de la souche non toxinogène de ribotype CE(032) permet de réduire de 89% le risque d'infections fatales associées à *Clostridioides difficile,* en particulier associées à la souche hypervirulente de ribotype PCR 027, par rapport à des patients n'ayant pas reçu préalablement la souche non toxinogène de ribotype CE(032) selon l'invention.

Dans un autre mode de réalisation particulièrement avantageux de l'invention, l'administration de manière préventive de la souche non toxinogène de ribotype CE(847) selon l'invention ou d'une composition comprenant au moins une souche non toxinogène de ribotype CE(847) selon l'invention comme seul principe actif permet de réduire de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque d'infections fatales associées à *Clostridioides difficile,* en particulier associées à la souche hypervirulente de ribotype PCR 027, par rapport à des patients n'ayant pas reçu préalablement la souche non toxinogène de ribotype CE(847) selon l'invention. Dans un mode de réalisation encore plus avantageux, l'administration de manière préventive de la souche non toxinogène de ribotype CE(847) selon l'invention ou d'une composition comprenant au moins une souche non toxinogène de ribotype CE(847) selon l'invention comme seul principe actif permet de réduire de 100% le risque d'infections fatales associées à *Clostridioides difficile,* en particulier associées à la souche hypervirulente de ribotype PCR 027, par rapport à des patients n'ayant pas reçu préalablement la souche non toxinogène de ribotype CE(847) selon l'invention.

Avantageusement, l'administration de manière préventive de la souche non toxinogène de ribotype CE(847) permet de réduire de 56% le risque d'infections fatales associées à *Clostridioides* difficile, en particulier associées à la souche hypervirulente de ribotype PCR 027, par rapport à des patients n'ayant pas reçu préalablement la souche non toxinogène de ribotype CE(847) selon l'invention.

Dans un autre mode de réalisation particulièrement avantageux de l'invention, l'administration de manière préventive d'un mélange de la souche non toxinogène de ribotype CE(032) et de la souche non toxinogène de ribotype CE(847) selon l'invention ou d'une composition comprenant un mélange de la souche non toxinogène de ribotype CE(032) et de la souche non toxinogène de ribotype CE(847) selon l'invention comme seul principe actif permet de réduire de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque d'infections fatales associées à *Clostridioides difficile,* en particulier associées à la souche hypervirulente de ribotype PCR 027, par rapport à des patients n'ayant pas reçu préalablement d'un mélange de la souche non toxinogène de ribotype CE(032) et de la souche non toxinogène de ribotype CE(847) selon l'invention. Dans un mode de réalisation encore plus avantageux, l'administration de manière préventive d'un mélange de la souche non toxinogène de ribotype CE(032) et de la souche non toxinogène de ribotype CE(847) selon l'invention ou d'une composition comprenant d'un mélange de la souche non toxinogène de ribotype CE(032) et de la souche non toxinogène de ribotype CE(847) selon l'invention comme seul principe actif permet de réduire de 100% le risque d'infections fatales associées à *Clostridioides difficile,* en particulier associées à la souche hypervirulente de ribotype PCR 027, par rapport à des patients n'ayant pas reçu préalablement d'un mélange de la souche non toxinogène de ribotype CE(032) et de la souche non toxinogène de ribotype CE(847) selon l'invention.

Dans un mode de réalisation particulièrement avantageux de l'invention, les risques de récidives des infections associées à *Clostridioides difficile* sont réduits de 50% lors de l'administration de manière préventive d'une souche non toxinogène de *Clostridioides difficile* selon l'invention ou d'une composition comprenant au moins une souche non toxinogène de *Clostridioides difficile* selon l'invention comme seul principe actif. Avantageusement, cette administration de manière préventive d'une souche non toxinogène de *Clostridioides difficile* selon l'invention ou d'une composition comprenant au moins une souche non toxinogène de *Clostridioides difficile* selon l'invention comme seul principe actif permet de réduire de plus de 50% le risque de récidives des infections associées à *Clostridioides difficile,* en particulier associées à la souche hypervirulente de ribotype PCR 027, par rapport à des patients n'ayant pas reçu préalablement au moins l'une des souches non toxinogènes selon l'invention.

Avantageusement, l'administration de manière préventive d'une souche non toxinogène de *Clostridioides difficile* selon l'invention ou d'une composition comprenant au moins une souche non toxinogène de *Clostridioides difficile* selon l'invention comme seul principe actif selon l'invention comme seul principe actif permet de réduire de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque de récidives des infections associées à *Clostridioides difficile,* en particulier associées à la souche hypervirulente de ribotype PCR 027, par rapport à des patients n'ayant pas reçu préalablement au moins l'une des souches non toxinogènes selon l'invention. Dans un mode de réalisation encore plus avantageux, l'administration de manière préventive d'une souche non toxinogène de *Clostridioides difficile* selon l'invention ou d'une composition comprenant au moins une souche non toxinogène de *Clostridioides difficile* permet de réduire de 100% le risque de récidives des infections associées à *Clostridioides difficile,* en particulier associées à la souche hypervirulente de ribotype PCR 027, par rapport à des patients n'ayant pas reçu préalablement au moins l'une des souches non toxinogènes selon l'invention.

Dans un mode de réalisation particulièrement avantageux de l'invention, l'administration de manière préventive de la souche non toxinogène de ribotype CE(032) selon l'invention ou d'une composition pharmaceutique comprenant la souche non toxinogène de ribotype CE(032) selon l'invention comme seul principe actif permet de réduire de plus de 50% avantageusement de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(032) selon l'invention. Dans un mode de réalisation encore plus avantageux, l'administration de manière préventive de la souche non toxinogène de ribotype CE(032) selon l'invention ou d'une composition pharmaceutique comprenant la souche non toxinogène de ribotype CE(032) selon l'invention comme seul principe actif permet de réduire de 100% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(032) selon l'invention.

Avantageusement, l'administration de manière préventive de la souche non toxinogène de ribotype CE(032) ou d'une composition pharmaceutique comprenant la souche non toxinogène de ribotype CE(032) selon l'invention comme seul principe actif permet de réduire de 89% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(032) selon l'invention.

Dans un autre mode de réalisation particulièrement avantageux de l'invention, l'administration de manière préventive de la souche non toxinogène de ribotype CE(847) selon l'invention ou d'une composition pharmaceutique comprenant la souche non toxinogène de ribotype CE(847) selon l'invention comme seul principe actif comme seul principe actif permet de réduire de plus de 50%, avantageusement de plus de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(847) selon l'invention. Dans un mode de réalisation encore plus avantageux, l'administration de manière préventive l'administration de manière préventive de la souche non toxinogène de ribotype CE(847) selon l'invention ou d'une composition pharmaceutique comprenant la souche non toxinogène de ribotype CE(847) selon l'invention comme seul principe actif permet de réduire de 100% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(847) selon l'invention.

Avantageusement, l'administration de manière préventive de la souche non toxinogène de ribotype CE(847) selon l'invention ou d'une composition pharmaceutique comprenant la souche non toxinogène de ribotype CE(847) selon l'invention comme seul principe actif permet de réduire de 56% le risque de récidives des infections associées à *Clostridioides difficile,* en particulier associées à la souche hypervirulente de ribotype PCR 027, par rapport à des patients n'ayant pas reçu préalablement la souche non toxinogène de ribotype CE(847) selon l'invention.

Dans un autre mode de réalisation particulièrement avantageux de l'invention, l'administration de manière préventive d'un mélange de la souche non toxinogène de ribotype CE(032) et de la souche non toxinogène de ribotype CE(847) selon l'invention ou d'une composition pharmaceutique comprenant d'un mélange de la souche non toxinogène de ribotype CE(032) et de la souche non toxinogène de ribotype CE(847) selon l'invention comme seul principe actif permet de réduire de plus de 50%, avantageusement de 55%, avantageusement de plus de 56%, avantageusement de plus de 57%, avantageusement de plus de 58%, avantageusement de plus de 59%, avantageusement de plus de 60%, avantageusement de plus de 61%, avantageusement de plus de 62%, avantageusement de plus de 63%, avantageusement de plus de 64%, avantageusement de plus de 65%, avantageusement de plus de 66%, avantageusement de plus de 67%, avantageusement de plus de 68%, avantageusement de plus de 69%, avantageusement de plus de 70%, avantageusement de plus de 71%, avantageusement de plus de 72%, avantageusement de plus de 73%, avantageusement de plus de 74%, avantageusement de plus de 75%, avantageusement de plus de 76%, avantageusement de plus de 77%, avantageusement de plus de 78%, avantageusement de plus de 79%, avantageusement de plus de 80%, avantageusement de plus de 81%, avantageusement de plus de 82%, avantageusement de plus de 83%, avantageusement de plus de 84%, avantageusement de plus de 85%, avantageusement de plus de 86%, avantageusement de plus de 87%, avantageusement de plus de 88%, avantageusement de plus de 89%, avantageusement de plus de 90%, avantageusement de plus de 91%, avantageusement de plus de 92%, avantageusement de plus de 93%, avantageusement de plus de 94%, avantageusement de plus de 95%, avantageusement de plus de 96%, avantageusement de plus de 97%, avantageusement de plus de 98%, avantageusement de plus de 99% le risque de récidives des infections associées à *Clostridioides difficile,* en particulier associées à la souche hypervirulente de ribotype PCR 027, par rapport à des patients n'ayant pas reçu préalablement d'un mélange de la souche non toxinogène de ribotype CE(032) et de la souche non toxinogène de ribotype CE(847) selon l'invention. Dans un mode de réalisation encore plus avantageux, l'administration de manière préventive d'un mélange de la souche non toxinogène de ribotype CE(032) et de la souche non toxinogène de ribotype CE(847) selon l'invention ou d'une composition pharmaceutique comprenant un mélange de la souche non toxinogène de ribotype CE(032) et de la souche non toxinogène de ribotype CE(847) selon l'invention comme seul principe actif permet de réduire de 100% le risque de récidives des infections associées à *Clostridioides difficile,* en particulier associées à la souche hypervirulente de ribotype PCR 027, par rapport à des patients n'ayant pas reçu préalablement d'un mélange de la souche non toxinogène de ribotype CE(032) et de la souche non toxinogène de ribotype CE(847) selon l'invention.

Un autre objet de l'invention concerne une méthode de traitement préventive des infections associées à *Clostridioides difficile* et/ou des récidives associées chez les patients, comprenant l'administration auxdits patients, d'une souche non toxinogène de *Clostridioides difficile* ou d'une composition pharmaceutique comprenant au moins une souche non toxinogène de *Clostridioides difficile* comme seul principe actif, telles que définies précédemment. Avantageusement, la souche non toxinogène de *Clostridioides difficile* est choisie parmi la souche non toxinogène de ribotype CE(032), la souche non toxinogène de ribotype CE(847) et un mélange de la souche non toxinogène de ribotype CE(032) et de la souche non toxinogène de ribotype CE(847).

Un autre aspect de l'invention concerne une souche non toxinogène de *Clostridioides difficile* selon l'invention ou une composition pharmaceutique comprenant au moins une souche non toxinogène de *Clostridioides difficile* comme seul principe actif destinée à être utilisée dans le traitement des infections associées à *Clostridioides difficile* et/ou les récidives associées.

Dans le cadre de l'invention, le terme « traitement » ou « traitement curatif » est défini par un traitement aboutissant à une guérison ou un traitement allégeant, améliorant et/ou éliminant, réduisant et/ou stabilisant les symptômes d'une maladie ou la souffrance qu'elle provoque.

Dans un mode de réalisation particulièrement avantageux, la présente invention concerne l'utilisation d'une composition pharmaceutique comprenant une quantité thérapeutiquement efficace d'au moins une souche non toxinogène de *Clostridioides difficile* telle que définie ci-dessus, en tant que seul principe actif et au moins un excipient pharmaceutiquement acceptable dans la fabrication d'un médicament destiné au traitement curatif des infections associées à *Clostridioides difficile* et/ou les récidives associées.

Dans un mode de réalisation particulièrement avantageux, la présente invention concerne l'utilisation d'une composition pharmaceutique comprenant une quantité thérapeutiquement efficace de la souche non toxinogène de ribotype CE(032) telle que définie ci-dessus, en tant que seul principe actif et au moins un excipient pharmaceutiquement acceptable dans la fabrication d'un médicament destiné au traitement curatif des infections associées à *Clostridioides difficile* et/ou les récidives associées.

Dans un mode de réalisation particulièrement avantageux, la présente invention concerne l'utilisation d'une composition pharmaceutique comprenant une quantité thérapeutiquement efficace de la souche non toxinogène de ribotype CE(847) telle que définie ci-dessus, en tant que seul principe actif et au moins un excipient pharmaceutiquement acceptable dans la fabrication d'un médicament destiné au traitement curatif des infections associées à *Clostridioides difficile* et/ou les récidives associées.

Dans un mode de réalisation particulièrement avantageux, la présente invention concerne l'utilisation d'une composition pharmaceutique comprenant une quantité thérapeutiquement efficace d'un mélange de la souche non toxinogène de ribotype CE(032) et de la souche non toxinogène de ribotype CE(847) tel que défini ci-dessus, en tant que seul principe actif et au moins un excipient pharmaceutiquement acceptable dans la fabrication d'un médicament destiné au traitement curatif des infections associées à *Clostridioides difficile* et/ou les récidives associées.

Selon l'invention, les compositions pharmaceutiques comprenant au moins une souche non toxinogène de *Clostridioides difficile* telles que définies précédemment sont particulièrement utiles pour traiter les diarrhées aigües ou chroniques et la colite pseudo-membraneuse, causées par *Clostridioides difficile* chez les patients.

Selon l'invention, les compositions pharmaceutiques comprenant au moins une souche non toxinogène de ribotype CE(032) telles que définies précédemment sont particulièrement utiles pour traiter les diarrhées aigües ou chroniques et la colite pseudo-membraneuse, causées par *Clostridioides difficile* chez les patients.

Selon l'invention, les compositions pharmaceutiques comprenant au moins une souche non toxinogène de ribotype CE(847) telles que définies précédemment sont particulièrement utiles pour traiter les diarrhées aigües ou chroniques et la colite pseudo-membraneuse, causées par *Clostridioides difficile* chez les patients.

Selon l'invention, les compositions pharmaceutiques comprenant au moins un mélange de la souche non toxinogène de ribotype CE(032) et de la souche non toxinogène de ribotype CE(847) telles que définies précédemment sont particulièrement utiles pour traiter les diarrhées aigües ou chroniques et la colite pseudo-membraneuse, causées par *Clostridioides difficile* chez les patients.

La présente invention permet aussi une diminution des effets indésirables par rapport aux antibiotiques actuellement utilisés en traitement, tels que le métronidazole, la vancomycine ou la fidaxomicine.

Dans un mode de réalisation particulièrement avantageux, la présente invention concerne l'utilisation d'une composition pharmaceutique comprenant une quantité thérapeutiquement efficace d'au moins une souche non toxinogène de *Clostridioides difficile* en tant que seul principe actif et au moins un excipient pharmaceutiquement acceptable, dans la fabrication d'un médicament destiné au traitement curatif des infections par *Clostridioides difficile* et/ou des récidives associées.

Dans un mode de réalisation particulièrement avantageux, la présente invention concerne l'utilisation d'une composition pharmaceutique comprenant une quantité thérapeutiquement efficace d'au moins une souche non toxinogène de ribotype CE(032) en tant que seul principe actif et au moins un excipient pharmaceutiquement acceptable, dans la fabrication d'un médicament destiné au traitement curatif des infections par *Clostridioides difficile* et/ou des récidives associées.

Dans un mode de réalisation particulièrement avantageux, la présente invention concerne l'utilisation d'une composition pharmaceutique comprenant une quantité thérapeutiquement efficace d'au moins une souche non toxinogène de ribotype CE(847) en tant que seul principe actif et au moins un excipient pharmaceutiquement acceptable, dans la fabrication d'un médicament destiné au traitement curatif des infections par *Clostridioides difficile* et/ou des récidives associées.

Dans un mode de réalisation particulièrement avantageux, la présente invention concerne l'utilisation d'une composition pharmaceutique comprenant une quantité thérapeutiquement efficace d'au moins un mélange de la souche non toxinogène de ribotype CE(032) et de la souche non toxinogène de ribotype CE(847) en tant que seul principe actif et au moins un excipient pharmaceutiquement acceptable, dans la fabrication d'un médicament destiné au traitement curatif des infections par *Clostridioides difficile* et/ou des récidives associées.

Un autre objet de l'invention concerne une méthode de traitement curative des infections associées à *Clostridioides difficile* et/ou des récidives associées chez les patients, comprenant l'administration auxdits patients, d'une souche non toxinogène de *Clostridioides difficile* ou d'une composition pharmaceutique comprenant au moins une souche non toxinogène de *Clostridioides difficile* comme seul principe actif, telles que définies précédemment. Avantageusement, la souche non toxinogène de *Clostridioides difficile* est choisie parmi la souche non toxinogène de ribotype CE(032), la souche non toxinogène de ribotype CE(847) et un mélange de la souche non toxinogène de ribotype CE(032) et de la souche non toxinogène de ribotype CE(847).

## FIGURES

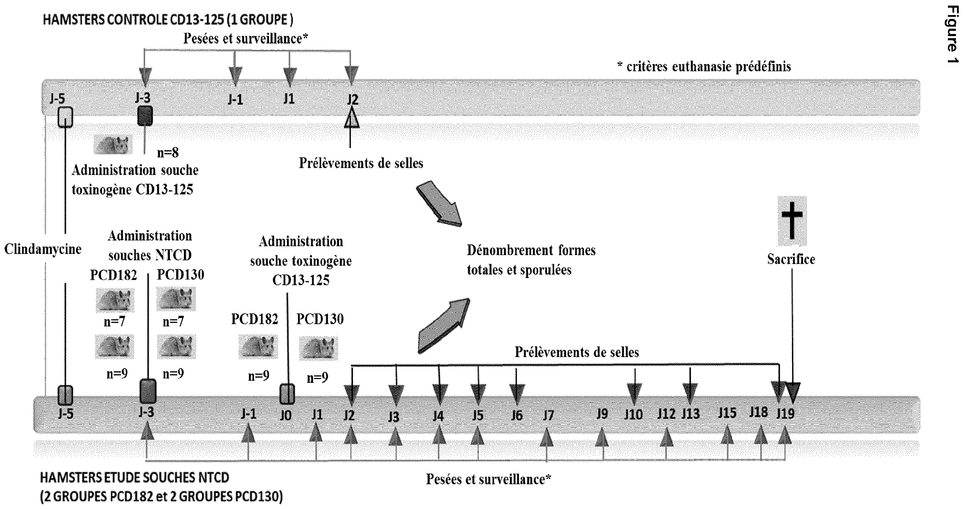

**Figure 1** : La Figure 1 représente le schéma expérimental de prévention des infections associées à *Clostridioides difficile* (ICD) par des souches non toxinogènes de ribotype CE(032) (également appelée NTCD PCD182) et CE(847) (également appelée NTCD PCD130), utilisées chez le hamster (n=40). Les hamsters ont été traités par clindamycine à Jour (J) -5. Les effectifs étant de n=8 hamsters pour le groupe contrôle, n=7 pour les groupes infectés par souches NTCD PCD182 et PCD130 seulement et n=9 pour les groupes inoculés par NTCD puis challengés par la souche toxinogène de ribotype 027 (également appelée CD13-125) à J0.

**Figure 2 :** La Figure 2 représente les valeurs des diamètres d'inhibition et des concentrations minimales inhibitrices (CMI) des antibiotiques testés.

**Figure 3** : La Figure 3 représente la courbe de survie des groupes de hamsters infectés avec les souches : CD13-125 (groupe A) ; PCD130 (groupe B) ; PCD130 et CD13-125 (groupe D) ; PCD182 (groupe C) ; PCD182 et CD13-125 (groupe E).

**Figure 4A** : La Figure 4A représente la cinétique du niveau de colonisation et du taux de hamsters colonisés dans le groupe CD13-125 (A), TOX=toxinogène ; NT / NTCD = non toxinogène.

**Figure 4B** : La Figure 4B représente la cinétique du niveau de colonisation et du taux de hamsters colonisés dans le groupe PCD130. TOX=toxinogène ; NT / NTCD = non toxinogène.

**Figure 4C** : La Figure 4C représente la cinétique du niveau de colonisation et du taux de hamsters colonisés dans le groupe PCD182 (C). TOX=toxinogène ; NT / NTCD = non toxinogène.

**Figure 4D** : La Figure 4D représente la cinétique du niveau de colonisation et du taux de hamsters colonisés dans le groupe PCD130+CD13-125. TOX=toxinogène ; NT / NTCD = non toxinogène.

**Figure 4E** : La Figure 4E représente la cinétique du niveau de colonisation et du taux de hamsters colonisés dans le groupe PCD182 (C) et PCD182+CD13-125. TOX=toxinogène ; NT / NTCD = non toxinogène.

**Figure 5** : La Figure 5 représente l'aspect macroscopique de caecum et de côlon. A gauche : hamster euthanasié suite à l'ICD (groupe D co-infecté PCD130 et CD13-125) ; A droite : hamster survivant à J19 sans signe clinique (groupe E co-infecté PCD182 et CD13-125).

**Figure 6** : La Figure 6 représente des exemples de coupes histologiques intestinales de hamsters survivants à J19 : en NTCD PCD130 (A) ; PCD182 (B); PCD130+CD13-125 (C) ; PCD182+CD13-125 (D). En E, coupe histologique intestinale de hamster euthanasié pour signes d'ICD dans le groupe PCD130+CD13-125.

**Figure 7 :** La Figure 7 représente la moyenne de la croissance bactérienne et de l'Absorbance en fonction du temps pour les souches NTCD PCD182 et PCD130 et toxinogène CD13-125. Trois expériences indépendantes ont été réalisées.

**Figure 8 :** La Figure 8 représente la cinétique du taux moyen de sporulation des souches NTCD PCD182, PCD130 et CD13-125 après traitement par l'alcool. Trois expériences indépendantes ont été réalisées.

**Figure 9 :** La Figure 9 représente le dénombrement à 24h (T24h) des formes totales en co-culture de la souche toxicogène CD13-125 avec les souches non toxinogènes PCD182 (A) et PCD 130(B) selon l'invention, selon différents ratios v/v (1/1 ; 5/1 ; 10/1) suivant deux conditions expérimentales.

## EXEMPLES

### Exemple 1 : Isolation et caractérisation des souches non toxinogènes de ribotype CE(032) et de ribotype CE(847).

Les deux souches non toxinogènes de ribotypes CE(032) et CE(847), appelées également NTCD PCD182 et NTCD PCD130 ont été isolées à partir de selles de nouveau-nés prématurés. La souche toxinogène utilisée dans le modèle de caecite du hamster est la souche CD13-25, isolée d'un patient souffrant d'ICD, hospitalisé en service de gériatrie à Marseille en Juin 2013. Cette souche appartient au ribotype 027 et est considérée comme hypervirulente.
Les souches, conservées à -80°C, ont été isolées sur géloses chromogéniques ChromID *C.difficile* (bioMérieux®). Les géloses ont été incubées 48H à 37°C en anaérobiose (enceinte anaérobie ou jarre anaérobie). À partir de la culture sur gélose, 10 ml de bouillon BHI ont été ensemencés et incubés 12h en anaérobiose. La suspension bactérienne a alors été utilisée pour les différentes expériences de caractérisation phénotypique.
La souche non toxinogène de *Clostridioides difficile* de ribotype CE(032) a été déposée le 12 février 2020 auprès du DSMZ (Leibniz Institute Deutsche Sammlung von Mikroorganismen und Zellkulturen GmbH) sous le numéro DSM 33438.
La souche non toxinogène de *Clostridioides difficile* de ribotype CE(847) a été déposée le 12 février 2020 auprès du DSMZ (Leibniz Institute Deutsche Sammlung von Mikroorganismen und Zellkulturen GmbH) sous le numéro DSM 33437.

### Dénombrement bactérien

Le dénombrement bactérien a été réalisé à partir des suspensions bactériennes et à partir des selles de hamster. Après prélèvement de 100 µl d'échantillon (culture *in vitro* ou prélèvement de suspension de selles) des dilutions sériées au 1/10ème réalisées dans de l'eau ou du PBS stérile sont ensemencées sur milieu gélosé et incubées 48h à 37°C en anaérobiose. Le nombre de colonies a été pris en compte si compris entre 10 et 300 colonies par gélose. Ce nombre a ensuite été reporté en UFC/ml en tenant compte de la dilution effectuée et du volume de l'inoculum ensemencé.

### PCR ribotypage

La caractérisation génétique des souches a été réalisée par PCR-ribotypage. Cette méthode de typage explore le polymorphisme des régions intergéniques (ou entretoises) situées entre l'ADN codant pour l'ARN 16S et 23S. Il s'agit de la méthode européenne de référence de typage des souches de *C. difficile*. Brièvement, l'extraction d'ADN s'est faite après prélèvement de quelques colonies sur gélose CLO (bioMérieux®) et mise en suspension dans 1 ml d'eau stérile. Après centrifugation (5 min à 13000 x rpm), le surnageant a été éliminé et le culot a été remis en suspension avec 200 µl de réactif pour extraction de l'ADN comme préconisé par le fournisseur (KIT InstaGene Matrix, Bio-Rad®). L'ADN extrait a été utilisé comme matrice pour la caractérisation par PCR-ribotypage (voir Tableau 1).

Tableau 1 : Conditions mises en œuvre pour le ribotypage par PCR

| Milieu | | | Conditions ribotypage PCR | | | |
|---|---|---|---|---|---|---|
| Réactif | Volume | Étape | Temps | Température | Cycles | |
| H2O stérile | 67μl | dénaturation | 4 min | 95°C | 1 | |
| Amorce CD1 | 1μl | Dénaturation / hybridation / élongation | 30 s | 94°C | 9 (-1°C par cycle) | |
| Amorce CD2 | 1μl | | 1 min | 65°C | | |
| dNTP | 10μl | | 1 min | 72°C | | |
| Tampon MgCl2 (Roche®) | 10μl | Dénaturation / hybridation / élongation | 30 s | 94°C | 30 | |
| Taq polymérase (Roche®) | 1μl | | 1 min | 57°C | | |
| Extrait ADN | 10μl | | 1 min | 72°C | | |

Les produits de PCR dilués au 1/10e ont été dénaturés à 90°C pendant 30s (Thermocycleur Verity 96, Applied Biosystem®) et la migration des fragments a été réalisée dans un Séquenceur 3500 Genetic Analyzer (Applied Biosystem®) à 8 capillaires, avec un polymère POP-7®. L'analyse des profils de migration a été réalisée grâce au logiciel GeneMapper (Applied Biosystem®) en comparant les profils obtenus avec ceux de la banque de données.

Exemple 2 : Effet de l'administration de manière préventive d'une souche non toxinogène (PCD182 ou PDC130) selon l'invention dans un modèle d'infection par *Clostridioides difficile* (ICD) chez le hamster

Préparation de l'inoculum initial de spores

À partir d'un isolement de chaque souche sur gélose CLO (bioMérieux®), incubée pendant 24h à 37°C en anaérobie, 3 colonies ont été prélevées, repiquées sur géloses COH (bioMérieux®) et incubées 48h à 37°C en anaérobiose. Les géloses ont ensuite été laissées à température ambiante pendant cinq jours, en aérobiose, afin de stimuler la production de spores. Après raclage des géloses au râteau, et mise en suspension des colonies dans 2 ml d'eau stérile, un choc alcoolique avec de l'alcool absolu (v/v, 30 min à 25°C) a été réalisé pour éliminer les éventuelles cellules végétatives restantes. Les spores ont alors été centrifugées (3000 x rpm, 20 min) et reprises dans 1 ml d'alcool absolu. Les tubes ont été passés 1 min au sonicateur (35MZ Sonorex SHE 10000, BANDELIN ®) afin de casser les agrégats et un état frais a été réalisé afin de confirmer la présence de spores. Les spores ont été purifiées par 2 lavages successifs avec du NaCl 0,9%, centrifugées (6000 x rpm, 5 min) et le culot final a été repris dans 700 μl de NaCl 0,9%. Le dénombrement des colonies sur les culots a ensuite été réalisé sur gélose ChromID *C. difficile* (BioMérieux®).

Les animaux

Quarante hamsters adultes de type « Syrian Aura », pesant entre 80 et 110 g (Laboratoires St Berthevin, France) ont été placés dans des cages individuelles pour éviter la contamination croisée entre les 3 souches de *C. difficile* étudiées, dès le début de l'expérience et pendant 19 jours. Les animaux ont été réceptionnés 13 jours avant le début du protocole et l'absence de contamination par *C. difficile* a été vérifiée avant le début de l'étude par culture des selles de chaque hamster sur milieu BHI 0,1% taurocholate.

Chaque animal a reçu une monodose de 50 mg/kg de clindamycine par gavage, afin de créer une dysbiose du microbiote intestinal à J-5. Deux jours plus tard, à J-3, les souches étudiées ont été administrées aux hamsters selon 5 groupes : un groupe contrôle positif auquel a été administré la souche toxinogène CD13-125 (n=8) ; deux groupes auxquels ont été administrées les souches NTCD PCD182 (n=7) et PCD130 seules (n=7) ; puis deux groupes auxquels ont été administrées les souches NTCD à J-3 suivies d'un challenge par la souche toxinogène 48h plus tard (à J0) (2 groupes de n=9).Les souches CD13-125, PCD182 et PCD130 ont été administrées par gavage (5.10$^3$ spores pour CD13-125 et 1.10$^6$ spores pour les souches NTCD). À partir de J-1, 24h après l'administration des souches NTCD (ou de la souche toxinogène pour le groupe contrôle), une surveillance quotidienne a été mise en place pour chacun des hamsters, pendant la durée de l'expérience, soit 19 jours, afin de détecter des manifestations cliniques d'ICD. Un score d'activité clinique d'activité de 0 à 2 a été utilisé (voir en particulier le Tableau 2).

Tableau 2 : Score clinique d'activité

| Score d'activité | Symptômes |
|---|---|
| 0 = euthanasie envisagée | Immobilité, bradypnée, ventre gonflé, « wet tail » et pattes mouillées, yeux plissés, poil hérissé, aréflexie |
| 1 | Ralentissement moteur, tremblements, ventre gonflé, tachypnée, poil hérissé, réaction aux stimuli externes, « wet tail », comportement d'isolement |
| 2 | Activité normale |

Les hamsters ont été pesés quotidiennement (à l'exception de certains dimanches ou jours fériés) de J-3 à J-19 afin de surveiller une éventuelle perte de poids. Des prélèvements de selles ont eu lieu à J-1 puis régulièrement, afin de suivre la cinétique de colonisation des souches (voir notamment la Figure 1).

Les critères d'euthanasie ont été définis au préalable et tout hamster présentant des signes cliniques d'ICD, une perte de poids de plus de 20g et un score clinique d'activité de 0 a été sacrifié. À J-19, tous les animaux survivants ont été sacrifiés et des prélèvements de caeca ont été réalisés. Les caecas ont été lavés et conservés dans une solution de PBS formolé 4% puis dans de l'éthanol 70% (+4°C) au moins 24h, avant d'être envoyés à la plateforme d'histologie de l'INRA (Institut National de Recherche Agronomique, Jouy-en-Josas). Après inclusion dans la paraffine et coupe au microtome (3 $\mu$m), les lames ont été colorées à l'hématoxyline et éosine avant d'être scannées et analysées à l'aide du logiciel 3DHISTECH Slide Converter ®.

Survie

Le décès de chaque animal a été enregistré afin de calculer un taux de survie pour chacun des groupes d'animaux.

PCR ribotypage des souches et contrôle

A chaque prélèvement de selles, 2 à 5 colonies isolées à partir des géloses destinées au dénombrement, ont été caractérisées par PCR-ribotypage, afin de confirmer la présence des souches obtenues dans chaque groupe d'animaux et d'exclure des contaminations croisées.

Résultats obtenus :

Les caractéristiques génétiques des souches non toxinogènes selon l'invention et de la souche toxinogène CD13-125 utilisées ont été vérifiées et confirmées (voir Tableau 3). Les deux souches NTCD appartiennent aux PCR ribotypes CE(032) et CE(847).

Tableau 3 : Souches et ribotypage PCR

| Souches | Ribotypage PCR |
|---|---|
| **PCD 182** (DSM 33438 déposée le 12 février 2020 auprès de la DMSZ) | **CE(032)** |
| **PCD 130** (DSM 33437 déposée le 12 février 2020 auprès de la DMSZ) | **CE(847)** |
| **CD13-25** | **K027** |

Pour chaque groupe d'animaux, le ribotypage des souches isolées à partir des prélèvements des selles a confirmé l'absence de contamination croisée entre animaux.

Cinétique de colonisation des souches en modèle animal : étude des formes totales et sporulées

Les selles des animaux ont été pesées puis suspendues dans 1 ml de tampon PBS. Après homogénéisation, 500 $\mu$l de cette suspension ont été traités par 500 $\mu$l d'éthanol absolu (30 min à 25°C) afin de sélectionner les formes sporulées. La suspension en PBS et celle traitée par l'alcool ont été énumérées par dilution sériée au 1/10e; 100 $\mu$l de chaque dilution (suspension PBS et suspension après traitement par l'alcool) ont été étalés sur gélose ChromID *C.difficile* pour les formes totales et sporulées. Pour les groupes de hamsters auxquels ont été administrées deux souches (NTCD et toxinogène), des milieux TECCA (Milieu TECCA : 52g de BHI Agar, 1g de Taurocholate, 2 ampoules de Supplément

*C. difficile* oxoid (cyclo/cefo) et 2mg d'érythromycine) ont également été ensemencés afin de sélectionner uniquement la souche toxinogène CD13-125 (résistance à l'érythromycine) et effectuer ainsi un dénombrement différentiel des 2 souches. Les milieux ensemencés ont été incubés pendant 48h à 37°C en anaérobie. Le résultat initialement en UFC/ml a été ensuite été exprimé en UFC/g de selles de l'animal.

### Caractérisation phénotypique des souches

### Sensibilité aux antibiotiques

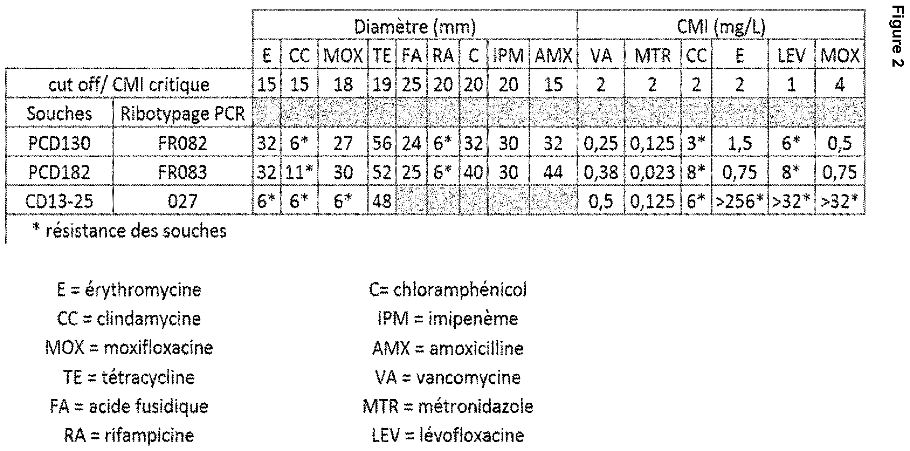

Des antibiogrammes ont été réalisés à partir de culture sur milieu BBA (bioMérieux®) ensemencés avec des inocula ajustés à 2 Mc Farland pour chaque souche puis incubés pendant 48h à 37°C en anaérobiose. Les antibiotiques testés sont présentés en Figure 2. Des bandelettes E-test (bioMérieux®) ont été utilisées pour déterminer la concentration minimale inhibitrice de chaque souche aux antibiotiques suivants vancomycine, métronidazole, érythromycine, moxifloxacine, lévofloxacine et clindamycine. Les niveaux de sensibilité aux antibiotiques ont été interprétés selon les *cut-off* (ou « valeurs seuils » en français) référencés par l'European Committee on Antimicrobial Susceptibility (EUCAST 2019) et le Comité de l'Antibiogramme de la Société Française de Microbiologie (2015)68.

### Cinétique de croissance bactérienne

À partir d'une suspension bactérienne en BHI liquide ajustée à une densité optique (DO) égale à 0,6, 100$\mu$l de suspension ont été inoculés dans 10 ml de BHI liquide. La mesure de l'absorbance a alors été réalisée au cours du temps toutes les 1,5h (de T0 à T10,5h) grâce à un spectrophotomètre (S 800 Diode Array Spectrophotometer, Biochrom®) pour PCD182, PCD130 et CD13-125. En parallèle aux mesures d'absorbance, le dénombrement bactérien a été réalisé.

### Sporulation bactérienne

100 $\mu$l d'une suspension bactérienne en milieu BHI liquide ont été inoculés dans 10 ml d'un milieu pré-réduit de sporulation SM (SM = Sporulation Médium : 90 g trypticase peptone, 5g de protéase peptone No3, 1 g de $(NH_4)_2SO_4$ et 1,5 g de Tris). Après incubation 72h, un dénombrement des formes totales (spores + formes végétatives) a été effectué à T0, T24, T36, T48 et T72h sur BHI 0,1% taurocholate. Pour chaque souche, la numération sélective de spores a été réalisée après choc alcoolique (mélange v/v dans de l'alcool absolu pendant 30 min à 25°C). Le traitement par l'alcool a pour but d'éliminer les formes végétatives pour ne garder que les spores. Les spores sont alors énumérées sur des milieux contenant du taurocholate qui permet de les faire germiner.

Le taux de sporulation a été déterminé selon la formule suivante :

$$Taux\ de\ sporulation = \frac{formes\ sporulées}{forme\ totale\ (= \ végétatives + sporulées)}\ x\ 100$$

### Co-culture des souches NTCD et de la souche CD13-125 en milieu liquide

A partir d'une suspension bactérienne en milieu BHI liquide, 100 $\mu$l de chaque culture ont été mis en culture dans 10 ml de BHI liquide et incubés 6h, selon les mêmes conditions, afin d'atteindre, pour chaque souche, la phase exponentielle de croissance bactérienne. Chaque culture a alors été centrifugée à 4300 x rpm pendant 20 min. Les culots bactériens ont été lavés avec du PBS et recentrifugés. Les culots obtenus ont alors été remis en suspension dans 5 ml de milieu BHI liquide puis 100 $\mu$l de cette suspension ont été ensemencés sur milieu BHI 0,1% taurocholate afin d'énumérer chaque souche à T0. Ensuite, 100 $\mu$l ont été prélevés afin de co-incuber chaque souche NTCD étudiée avec la souche toxinogène CD13-125 selon différents ratios (ratio souche NTCD/ souche toxinogène : 1/1 ; 5/1 et 10/1). Les co-cultures et les contrôles contenant chaque souche seule ont été incubés 24h en anaérobiose à 37°C. À T24h, 100 $\mu$l ont été prélevés et un dénombrement des colonies a été réalisé pour les formes totales et sporulées. La numération de chaque souche mise en co-culture s'est faite grâce à l'ensemencement simultané de gélose BHI 0,1% taurocholate et BHI 0,1% taurocholate supplémentée en érythromycine (52g BHI Agar, 1g de Taurochlorate et 2mg d'érythromycine) qui permet la sélection de la souche CD13-125.

### Croissance de *C. difficile* toxinogène en présence du surnageant des souches NTCD

Cette technique a pour but de voir si les surnageants de culture des souches NTCD ont un effet inhibiteur sur la croissance de la souche CD13-125. Pour cela, 15 $\mu$l des surnageants de culture des souches NTCD PCD182 et PCD130 ont été transférés sur des disques stériles, apposés sur des géloses BHI 0,1% taurocholate ensemencées au préalable par une suspension de la souche CD13-125 ajustée à 0,5 Mc Farland. L'effet inhibiteur des surnageants a été observé en mesurant le diamètre d'inhibition après 48h d'incubation à 37°C en anaérobiose.

Statistiques

Les pentes des courbes de croissances des souches ont été comparées grâce à un test de Fisher de régression linéaire (GraphPad Prism®, San Diego, USA). Un test de Fisher exact a permis de comparer les taux de survie des animaux à J-19 entre les groupes traités. Un test du log-rank a été utilisé afin de comparer les fonctions de survie entre les groupes, avec correction de Benjamini Hochberg ou FDR (survival package, Rstudio®). Le risque $\alpha$ a été considéré acceptable à 5% pour l'ensemble des tests.

Résultats

Survie des animaux

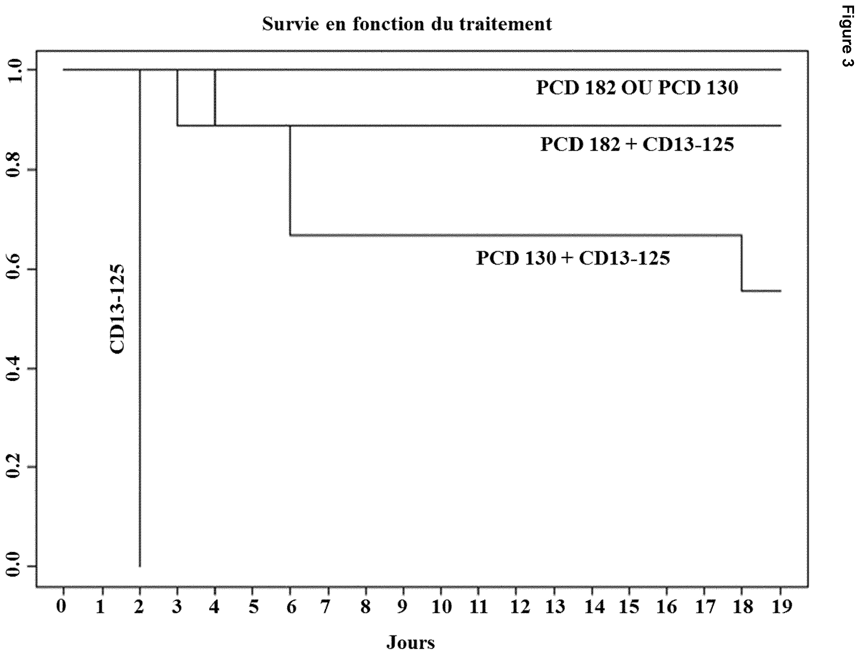

La souche toxinogène CD13-125 a entraîné le décès de 100% des hamsters du groupe A (groupe contrôle positif de l'ICD) au bout de 48h (Figure 3). Dans les deux groupes de hamsters auxquels ont été administrées les souches NTCD PCD130 (groupe D) et PCD182 (groupe E), les taux de mortalité étaient respectivement de 44,4% (4/9 hamsters) et 11,1% (1/9 hamsters) à J19. Les 4 hamsters décédés du groupe D, co-infectés par les souches NTCD PCD130 et toxinogène CD13-125 ont été euthanasiés car ils présentaient des scores d'activité bas (signes cliniques spécifiques « wet-tail » et selles liquides), et une perte de poids significative d'environ 20g. Le hamster décédé du groupe E co-infecté par les souches NTCD PCD182 et toxinogène CD13-125 est mort sans que les signes cliniques aient été détectés.
La comparaison des taux de survie à J19 montre une amélioration significative du taux de survie des animaux en présence de la souche PCD130 (groupe D) (p=0,029) ou de la souche NTCD PCD182 (groupe E) (p=0,0004) par rapport au groupe infecté par la souche toxinogène CD13-125 (groupe A). La comparaison des taux de survie à J19 entre les groupes D et E (souches NTCD) ne montre pas de différence significative (p=0,29). Les probabilités de survie (test du log rank) sont significativement différentes si l'on considère chaque groupe traité par souche NTCD (groupes D et E) *vs* le groupe contrôle d'ICD (groupe A) (p=0,00006 dans les deux cas).

Étude de la colonisation par les formes totales et sporulées des souches bactériennes

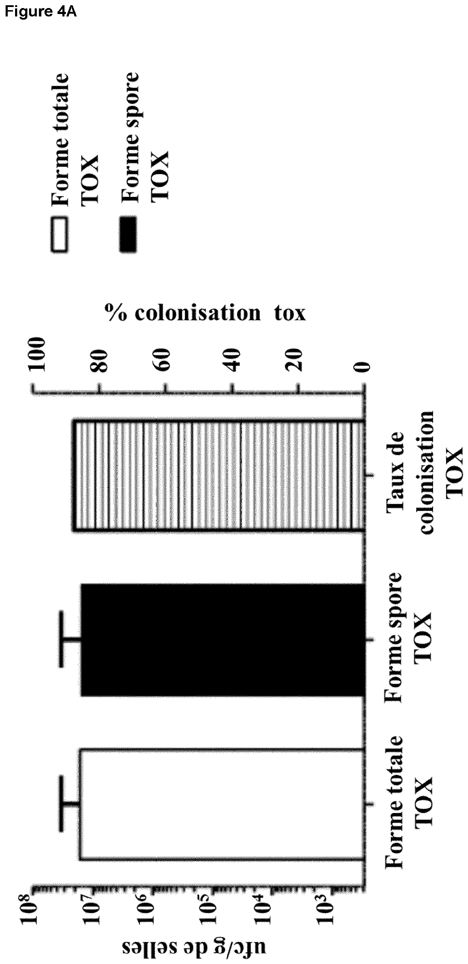

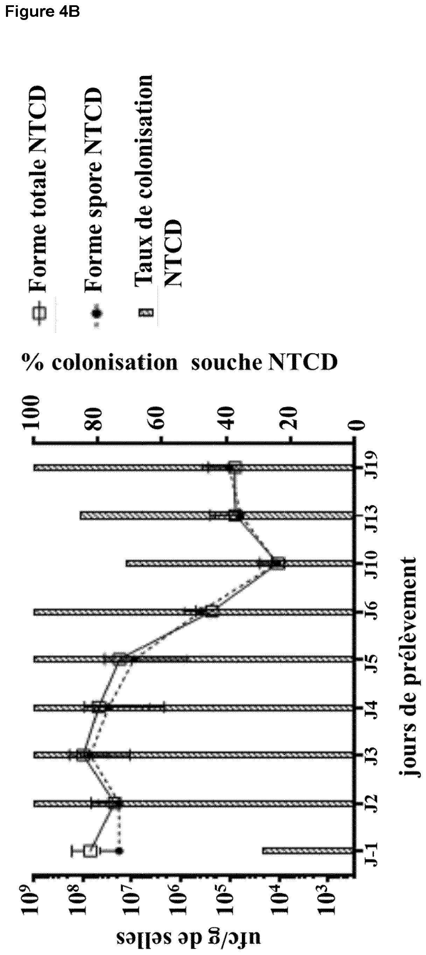

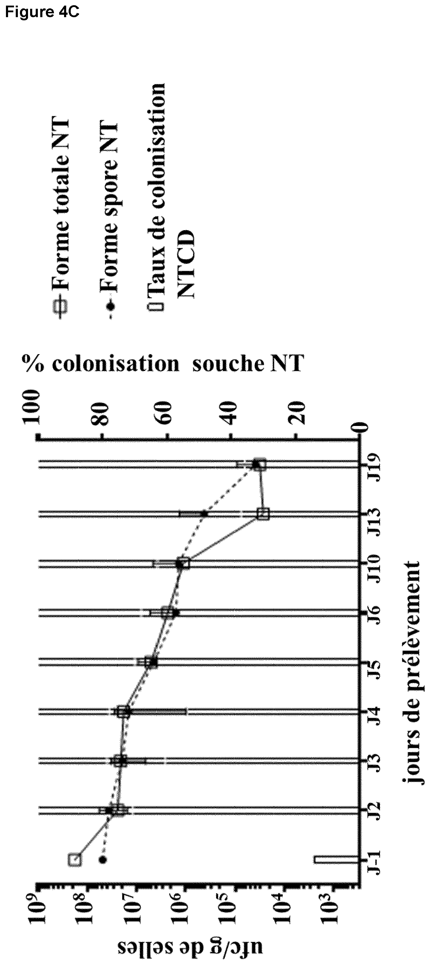

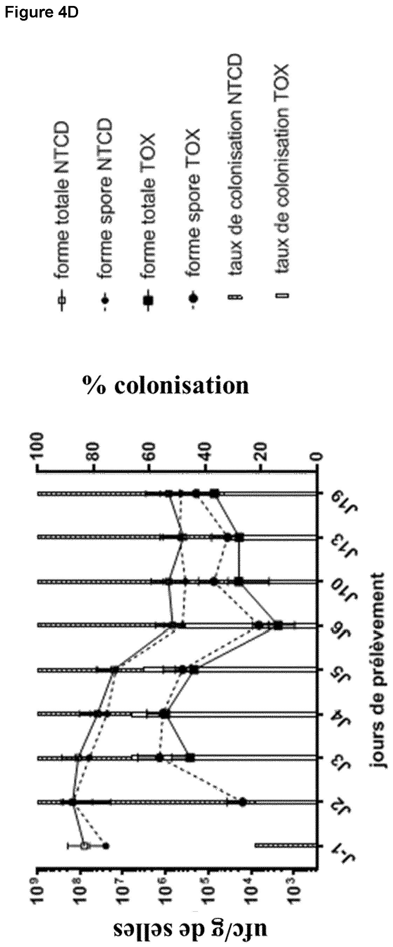

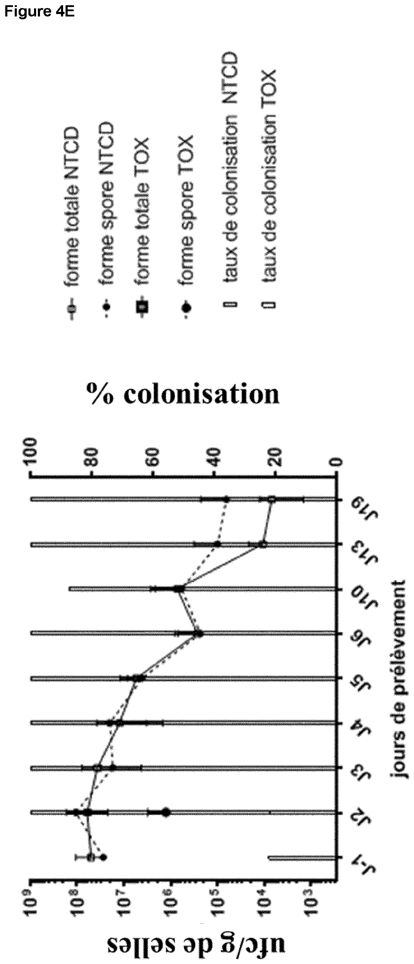

Les hamsters du groupe A infectés par la souche CD13-125 toxinogène a montré à J2 un niveau de colonisation moyen de $1,6.10^7$ UFC/g de selles (93% de spores). Ce taux est équivalent aux autres groupes d'animaux.
A J2, les niveaux de colonisation pour les formes totales (végétatives et sporulées) dans les deux groupes traités par souche NTCD PCD130 (groupes B et D) étaient de $2,3.10^7$ et $1,5.10^8$ UFC/g de selles (Figures 4A à 4E). Les formes sporulées à J2 pour ces deux groupes s'élevaient en moyenne à 81% et 98% de la population bactérienne totale. Dans les deux autres groupes, auxquels a été administrée la souche NTCD PCD182 (groupes C et E), les formes totales ont été dénombrées à $2,4.10^7$ et $6,2.10^7$ UFC/g de selles, les spores représentaient en moyenne 100% de la population bactérienne pour ces deux groupes. Entre J2 et J19, on observe une diminution régulière du niveau de colonisation que ce soit dans les groupes d'animaux colonisés par les souches NTCD seules ou dans les groupes d'animaux co-infectés avec la souche toxinogène.
A J19, les hamsters survivants ont montré des taux de colonisation moyens par les formes totales des souches NTCD entre $10^3$ et $10^5$ UFC/g de selles. Pour les groupes B et D (NTCD PCD130), on retrouve $7,9.10^4$ et $8,5.10^5$ UFC/g de selles et pour les groupes C et E (NTCD PCD182) les niveaux sont respectivement de $3,1.10^4$ et $6,9.10^3$ UFC/g de selles. Les niveaux de spores à J19 sont de 100% et 51% pour les groupes traités par la souche PCD130 et à 100% pour les deux groupes traités par la souche PCD182.
Dans les groupes co-infectés, 4 hamsters sur 9 ont montré une colonisation par la souche toxinogène perdurant dans le temps malgré l'administration de la souche NTCD : les niveaux de colonisation chez ces hamsters varient entre $10^4$ et $10^6$ UFC/g de selles, conduisant aux symptômes de l'ICD avec pour conséquence soit la mort ou l'euthanasie. Les taux de formes sporulées de CD13-125 retrouvés chez ces animaux sont proches de 100%.
Dans le groupe E, la colonisation par CD13-125 n'a été observée que sur 2 hamsters sur 9, uniquement à J2 et sous forme sporulée. Aucune colonisation par CD13-125 n'a été mise en évidence jusqu'à J19 sur les prélèvements de selles des hamsters survivants.

Aspect nécrosique et analyse histologique

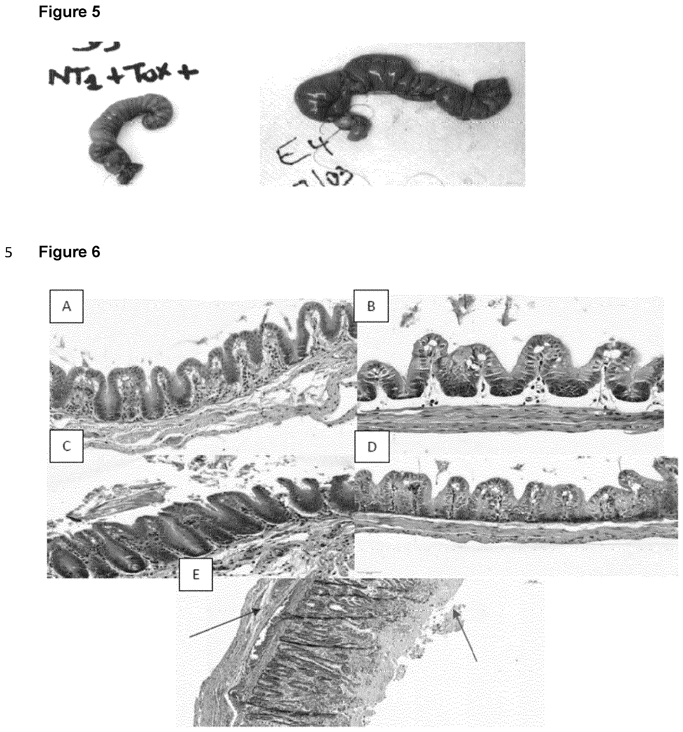

Les caecas et colons des hamsters décédés du groupe contrôle ainsi que le hamster décédé dans le groupe co-infecté par la souche NTCD PCD182 et CD13-125, n'ont pu être prélevés à cause de la rigidité des tissus. L'aspect caractéristique hémorragique et hyperplasique des organes prélevés sur les hamsters euthanasiés suite à l'apparition de signes cliniques d'ICD est présenté en Figure 5.

Parmi les animaux prélevés post-sacrifice à J19, les hamsters inoculés avec les souches NTCD ont présenté une absence d'altération tissulaire, ainsi que les hamsters des groupes co-infectés survivants sans signes cliniques. Parmi les groupes co-infectés, les hamsters euthanasiés suite aux signes cliniques d'ICD, ont présenté une altération tissulaire caecale importante avec hémorragies diffuses, infiltration par des cellules pro-inflammatoires (polynucléaires neutrophiles), perte de l'architecture cryptique intestinale, hyperplasie et desquamation épithéliale (Figure 6).

## Évolution du poids des animaux

La cinétique de l'évolution du poids des animaux montre une augmentation du poids des animaux dans tous les groupes, excepté chez les hamsters du groupe contrôle d'ICD ou décédés précocement pour les groupes co-infectés présentant les signes cliniques d'ICD.

## Exemple 3 : Caractérisation phénotypique des souches de ribotype CE(847) (PCD130) et CE(032) (PCD182)

### Sensibilité des souches aux antibiotiques

Le niveau de sensibilité aux antibiotiques de chaque souche est présenté en Figure 2. Les souches NTCD PCD130 et PCD182 ont un profil semblable avec une résistance vis-à-vis de la rifampicine, la clindamycine et la lévofloxacine. La souche toxinogène CD13-125, est résistante à l'érythromycine, à la clindamycine, et aux 2 fluoroquinolones (moxifloxacine et lévofloxacine). Le fait que la souche toxinogène soit résistante à l'érythromycine, nous a permis d'utiliser ce marqueur pour la numération différentielle des souches NTCD et toxinogène pour les expériences incluant des dénombrements bactériens.

### Croissance bactérienne

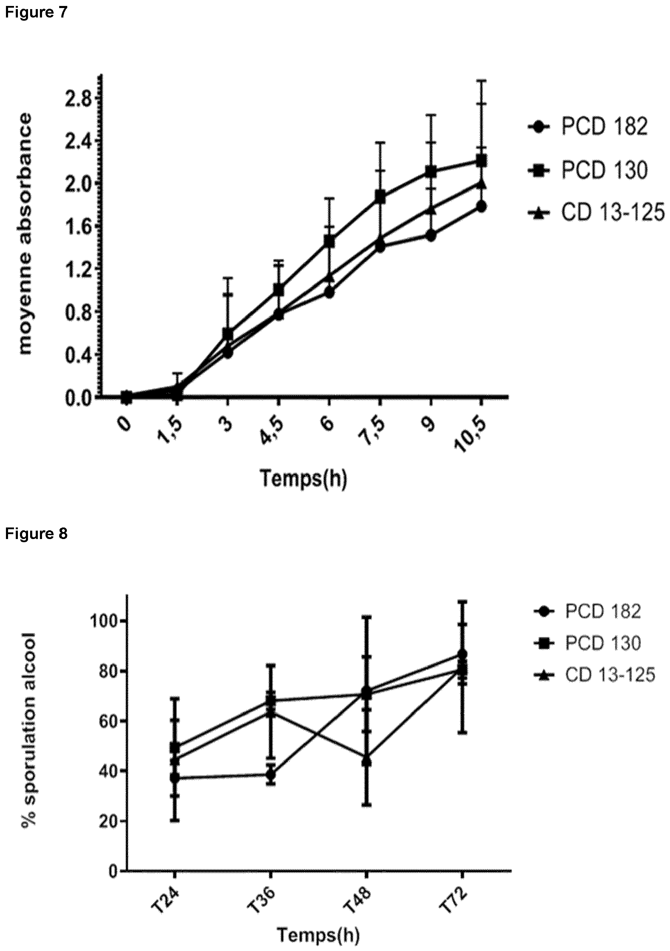

Les courbes de croissance des trois souches sont représentées dans la Figure 7. Pour toutes les souches, la phase exponentielle de croissance se situe aux alentours de 6 heures (DO = 1). La comparaison des différents points des courbes de croissance des souches ne montre pas de différence significative entre la souche PCD130 et la souche CD13-125 toxinogène que ce soit en termes d'absorbance (p=0,53) ou de dénombrement des colonies (p=0,18) ; il en est de même pour la souche NTCD PCD182 et la souche toxinogène (respectivement p=0,31 et p=0,58).

### Sporulation des souches bactériennes

Les résultats de la cinétique de sporulation des différentes souches sont présentés en Figure 8. Le traitement à l'alcool montre des profils de sporulation équivalents avec un taux moyen de sporulation d'environ 80% après 72h. La souche PCD182 semble avoir un léger retard à la sporulation à 36 heures par rapport aux deux autres souches

### Co-culture des souches bactériennes en milieu liquide

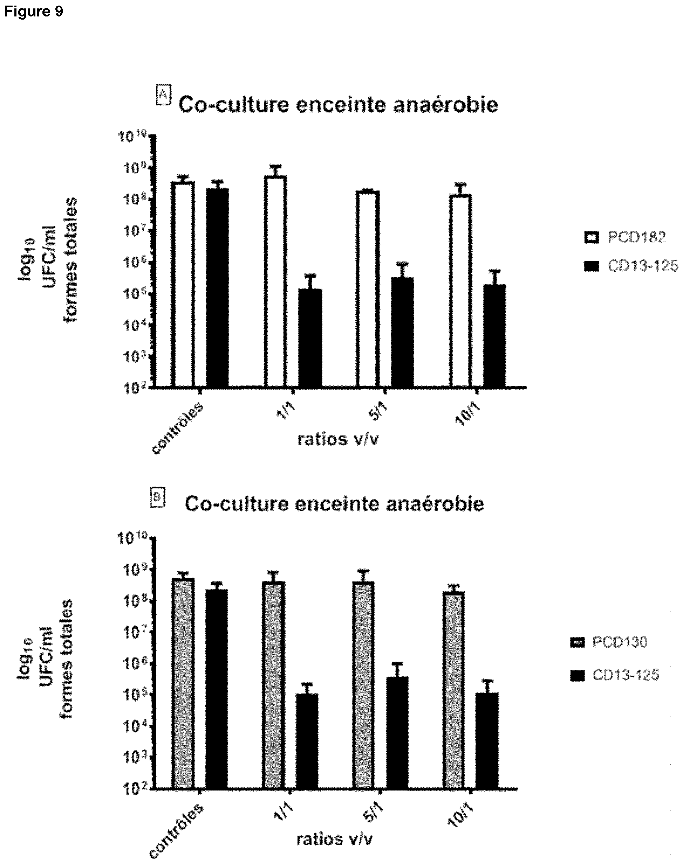

Afin d'observer si les souches NTCD PCD130 et PCD182 étaient capables d'inhiber la croissance de *C. difficile* toxinogène CD13-125 de ribotype 027, un système de co-culture en milieu liquide a été utilisé. Les résultats sont présentés en Figure 9. Il s'agit de données préliminaires, une expérience seulement pour chaque condition ayant été réalisée. Les résultats montrent que, quel que soit le ratio de mélange des souches, les 2 souches NTCD inhibent drastiquement la croissance de la souche toxinogène CD13-125.

### Croissance de *C. difficile* toxinogène en présence du surnageant des souches NTCD

Une souche de *C. difficile* toxinogène a été incubée avec les disques imprégnés des surnageants de culture de chaque souche NTCD PCD130 et PCD182, pendant 48h, à 37°C et dans des conditions d'anaérobiose. Les résultats montrent que les surnageants de culture des souches NTCD PCD130 et PCD182 ne permettent pas d'inhiber la croissance de la souche toxinogène.

### Typage des souches par Multi-Locus Sequence Typing (MLST)

Les souches NTCD PCD130 et PCD182 ont été caractérisées par la technique de MLST suivant le protocole décrit par Griffiths et al (Griffiths D, et al. J Clin Microbiol. 2010 Mar;48(3):770-8). Les séquences types (ST) des souches sont présentées dans le tableau 4.

Tableau 4 : Souches et séquence type (ST) par MLST

| Souches | adk | atp A | dxr | gly A | rec A | sodA | tpi | ST | clade | PCR-ribotype |
|---------|-----|-------|-----|-------|-------|------|-----|-----|-------|--------------|
| PCD 130 | 1 | 1 | 6 | 1 | 4 | 3 | 4 | 26 | 1 | (CE)847 |
| PCD 182 | 1 | 1 | 6 | 1 | 1 | 3 | 1 | 83 | 1 | (CE)032 |

**Revendications**

1. Souche non toxinogène de *Clostridioides difficile* pour son utilisation dans la prévention primaire et/ou le traitement des infections associées à *Clostridioides difficile* et/ou des récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847).

2. Souche non toxinogène de *Clostridioides difficile* selon la revendication 1, dans laquelle la souche non toxinogène de *Clostridioides difficile* est isolée à partir de selles de nouveau-nés prématurés.

3. Souche non toxinogène de *Clostridioides difficile* selon l'une des revendications 1 à 2, dans laquelle la souche non toxinogène de ribotype CE(032) a été déposée le 12 février 2020 auprès du DSMZ (Leibniz Institute Deutsche Sammlung von Mikroorganismen und Zellkulturen GmbH) sous le numéro DSM 33438.

4. Souche non toxinogène de *Clostridioides difficile* selon l'une des revendications 1 à 2, dans laquelle la souche non toxinogène de ribotype CE(847) a été déposée le 12 février 2020 auprès du DSMZ (Leibniz Institute Deutsche Sammlung von Mikroorganismen und Zellkulturen GmbH) sous le numéro DSM 33437.

5. Composition pharmaceutique comprenant au moins une souche non toxinogène de *Clostridioides difficile* comme principe actif, pour son utilisation dans la prévention primaire et/ou le traitement des infections associées à *Clostridioides difficile* et/ou récidives associées, ladite souche non toxinogène de *Clostridioides difficile* étant choisie parmi le groupe comprenant la souche non toxinogène de ribotype CE(032), la souche non toxinogène de ribotype CE(847) et un mélange des souches non toxinogènes de ribotype CE(032) et de ribotype CE(847).

6. Composition pharmaceutique selon la revendication 5, dans laquelle la souche non toxinogène de ribotype CE(032) a été déposée le 12 février 2020 auprès du DSMZ (Leibniz Institute Deutsche Sammlung von Mikroorganismen und Zellkulturen GmbH) sous le numéro DSM 33438 et la souche non toxinogène de ribotype CE(847) a été déposée le 12 février 2020 auprès du DSMZ (Leibniz Institute Deutsche Sammlung von Mikroorganismen und Zellkulturen GmbH) sous le numéro DSM 33437.

7. Composition pharmaceutique selon l'une des revendications 5 à 6, dans laquelle la composition comprend une quantité thérapeutiquement efficace de souche non toxinogène de *Clostridioides difficile.*

8. Composition pharmaceutique selon la revendication 7, dans laquelle la composition pharmaceutique comprend au moins $10^4$ spores de souche non toxinogène de *Clostridioides difficile.*

9. Composition pharmaceutique selon l'une des revendications 5 à 8, comprenant en outre au moins un excipient pharmaceutiquement acceptable.

10. Composition pharmaceutique selon l'une des revendications 5 à 9, dans laquelle la composition pharmaceutique est sous une forme adaptée pour son administration par voie orale et/ou sous forme de compléments alimentaires et/ou produits alimentaires.

Figure 1

Figure 2

EP 3 871 683 A1

| | | Diamètre (mm) | | | | | | | | CMI (mg/L) | | | | | |
|---|---|---|---|---|---|---|---|---|---|---|---|---|---|---|---|
| | | E | CC | MOX | TE | FA | RA | C | IPM | AMX | VA | MTR | CC | E | LEV | MOX |
| cut off/ CMI critique | | 15 | 15 | 18 | 19 | 25 | 20 | 20 | 20 | 15 | 2 | 2 | 2 | 2 | 1 | 4 |
| Souches | Ribotypage PCR | | | | | | | | | | | | | | | |
| PCD130 | FR082 | 32 | 6* | 27 | 56 | 24 | 6* | 32 | 30 | 32 | 0,25 | 0,125 | 3* | 1,5 | 6* | 0,5 |
| PCD182 | FR083 | 32 | 11* | 30 | 52 | 25 | 6* | 40 | 30 | 44 | 0,38 | 0,023 | 8* | 0,75 | 8* | 0,75 |
| CD13-25 | 027 | 6* | 6* | 6* | 48 | | | | | | 0,5 | 0,125 | 6* | >256* | >32* | >32* |

* résistance des souches

E = érythromycine

CC = clindamycine

MOX = moxifloxacine

TE = tétracycline

FA = acide fusidique

RA = rifampicine

C= chloramphénicol

IPM = imipenème

AMX = amoxicilline

VA = vancomycine

MTR = métronidazole

LEV = lévofloxacine

Survie en fonction du traitement

PCD 182 OU PCD 130

PCD 182 + CD13-125

PCD 130 + CD13-125

CD13-125

Jours

Figure 3

EP 3 871 683 A1

26

**Figure 4A**

Figure 4B

EP 3 871 683 A1

Figure 4C

Figure 4D

**Figure 4E**

Figure 5

Figure 6

**Figure 7**

**Figure 8**

Figure 9

A  Co-culture enceinte anaérobie

PCD182
CD13-125

B  Co-culture enceinte anaérobie

PCD130
CD13-125

Europäisches Patentamt

European Patent Office

Office européen des brevets

## RAPPORT DE RECHERCHE EUROPEENNE

Numéro de la demande

EP 21 15 8882

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | SEAL D ET AL: "TREATMENT OF RELAPSING CLOSTRIDIUM DIFFICILE DIARRHOEA BY ADMINISTRATION OF A NON-TOXIGENIC STRAIN", EUROPEAN JOURNAL OF CLINICAL MICROBIOLOGY & INFECTIOUS DISEASES, SPRINGER, WIESBADEN, DE, vol. 6, no. 1, 1 février 1987 (1987-02-01), pages 51-53, XP000985052, ISSN: 0934-9723, DOI: 10.1007/BF02097191 * pages 51-52 * | 1-10 | INV. A61K35/742 A61P1/12 A61K9/00 A23L33/135 |
| Y | BORRIELLO S P ET AL: "PROTECTION OF HAMSTERS AGAINST CLOSTRIDIUM DIFFICILE ILEOCAECITIS BY PRIOR COLONISATION WITH NON-PATHOGENIC STRAINS", JOURNAL OF MEDICAL MICROBIOLOGY, CHURCHILL LIVINGSTONE [ETC.], vol. 19, 1 janvier 1985 (1985-01-01), pages 339-350, XP002945324, ISSN: 0022-2615 * pages 343-344 * | 1-10 | |
| | ----- | | DOMAINES TECHNIQUES RECHERCHES (IPC) |
| Y | SANDRA JANEZIC ET AL: "International Clostridium difficile animal strain collection and large diversity of animal associated strains", BMC MICROBIOLOGY, BIOMED CENTRAL LTD, GB, vol. 14, no. 1, 28 juin 2014 (2014-06-28), page 173, XP021190035, ISSN: 1471-2180, DOI: 10.1186/1471-2180-14-173 * tableau 1 * | 1-10 | A61K A61P A23L |
| | ----- | | |
| | -/-- | | |

Le présent rapport a été établi pour toutes les revendications

| Lieu de la recherche | Date d'achèvement de la recherche | Examinateur |
|---|---|---|
| Munich | 14 juin 2021 | Bochelen, Damien |

EPO FORM 1503 03.82 (P04C02)

page 1 de 2

Europäisches Patentamt
European Patent Office
Office européen des brevets

## RAPPORT DE RECHERCHE EUROPEENNE

Numéro de la demande

EP 21 15 8882

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | LAURENT FERRARIS ET AL: "Carriage and colonization of C. difficile in preterm neonates: A longitudinal prospective study", PLOS ONE, vol. 14, no. 2, 20 février 2019 (2019-02-20), page e0212568, XP055738811, DOI: 10.1371/journal.pone.0212568 * page 5; tableau 2 * ----- | 1-10 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 14 juin 2021 | Bochelen, Damien |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

                                               
& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **VILLANO, S. A. ; SEIBERLING, M. ; TATAROW-ICZ, W. ; MONNOT-CHASE, E. ; GERDING, D. N.** . Evaluation of an Oral Suspension of VP20621, Spores of Nontoxigenic Clostridioides difficile Strain M3, in Healthy Subjects. *Antimicrob. Agents Chemother.,* 2012, vol. 56, 5224-5229
- **GERDING, D. N. et al.** Administration of Spores of Nontoxigenic Clostridioides difficile Strain M3 for Prevention of Recurrent C difficile Infection: A Randomized Clinical Trial. *JAMA,* 2015, vol. 313, 1719

- *l'European Committee on Antimicrobial Susceptibility,* 2019
- *Comité de l'Antibiogramme de la Société Française de Microbiologie,* 2015, 68
- **GRIFFITHS D et al.** *J Clin Microbiol.,* Mars 2010, vol. 48 (3), 770-8